# EUROPÄISCHE PATENTSCHRIFT

(11) **EP 2 852 423 B1**
(45) Veröffentlichungstag und Bekanntmachung des Hinweises auf die Patenterteilung: **11.07.2018**
(21) Anmeldenummer: 13724241.8
(22) Anmeldetag: 17.05.2013
(51) Int. Cl.: A61M 15/00, A61K 9/48, A61K 9/00, A61J 3/07, B65D 47/28

(54) **SYSTEM AUS INHALATOR UND KAPSEL**
INHALER AND CAPSULE FOR AN INHALER
INHALATEUR ET CAPSULE POUR INHALATEUR

(30) Priorität: 21.05.2012 WO PCT/EP2012/059324
(43) Veröffentlichungstag der Anmeldung: 01.04.2015
(73) Patentinhaber: Boehringer Ingelheim International GmbH, 55216 Ingelheim am Rhein (DE)
(72) Erfinder: HOLAKOVSKY, Holger, 55216 Ingelheim am Rhein (DE); FRENTZEL-BEYME, Jessica, 55216 Ingelheim am Rhein (DE); DUNNE, Stephen, Terence, Europark Ipswich IP3 9BF (GB); BESSELER, Jens, 55216 Ingelheim am Rhein (DE); WEILER, Claudius, 55216 Ingelheim am Rhein (DE)
(74) Vertreter: Simon, Elke Anna Maria
(86) Internationale Anmeldenummer: PCT/EP2013/060272
(87) Internationale Veröffentlichungsnummer: WO 2013/174752

(56) Entgegenhaltungen:
- WO-A1-01/72605
- DE-A1-102004 040 928
- FR-A- 1 215 560
- FR-A2- 2 032 436
- US-A- 4 076 848
- US-A1- 2004 131 668
- US-A1- 2004 173 211
- US-A1- 2009 194 105

## Beschreibung

Die vorliegende Erfindung betrifft ein System aus einer Kapsel zur Aufnahme von medizinischen Formulierungen zum Einsatz in einem Inhalator und einem zugehörigen Inhalator. Insbesondere bezieht sich die Erfindung auf Systeme mit Kapseln, die mit einer pulverförmigen pharmazeutischen Zubereitung gefüllt sind und auf Inhalatoren, mit denen eine pulverförmige pharmazeutische Zubereitung zur Inhalation bereit gestellt werden soll, wobei sich das Pulver in einer Kapsel befindet und zur Inhalation mittels mindestens eines Lochs in der Kapselwand aus der Kapsel austritt.

Aus dem Stand der Technik sind Kapseln bekannt, die in bestimmten medizinischen Vorrichtungen wie Pulverinhalatoren zum Einsatz kommen. Die äußere Form von in solchen Inhalatoren verwendeten Kapseln ist häufig (wie auch in der vorliegenden Schrift) die eines geschlossenen Zylinders mit halbkugelförmigen Enden, wobei die Länge des Zylinders größer ist als sein Durchmesser. Solche Kapseln bestehen üblicherweise aus zwei becherartigen Teilen, nämlich einem Kapselkörper und einer Kapselkappe, die teleskopartig ineinander gesteckt sind. Verschiedene Materialien solcher Kapseln sind bekannt. Viele in der Medizin verwendete Kapseln bestehen aus Gelatine oder Hartgelatine.
Die WO2000/07572 offenbart Kunststoffkapseln zur Verwendung in Pulverinhalatoren. Die Kapseln bestehen aus Kapselkörper und Kapselkappe, die so miteinander verbunden werden können, dass ein stabiler, abgeschlossener Hohlraum von definiertem Volumen gebildet wird. Die Kapsel kann Rastelemente aufweisen, welche die Kapselkappe fest mit dem Kapselkörper verbinden. Ein Beispiel für solche Rastelemente sind punktförmige Erhebungen im Innenmantel der Kapselkappe, die in etwas größere punktförmige Vertiefungen am Außenmantel des Kapselkörpers einrasten. Kapselkappe und Kapselkörper bestehen beide aus dem gleichen nicht-wasserlöslichen, hydrophoben Kunststoff, vorzugsweise Polyethylen.
Die WO2006/074982 A2 offenbart ein Verschlusskonzept für Kapselkappe und Kapselkörper, durch dass es möglich ist, die beiden Teile für den Transport der Kapsel zur Befüllanlage provisorisch über einen Vorverschluss zu verbinden, der im Gegensatz zum Hauptverschluss zerstörungsfrei geöffnet werden kann. Die Verschlüsse werden im Innenmantel der Kapselkappe durch ringförmig verlaufende oder segmentartige Erhebungen und dazu passende peripher den Außenmantel des Kapselkörpers ringförmig umlaufende Vertiefungen gebildet.

Aus dem Stand der Technik sind verschiedene Pulverinhalatoren bekannt, bei denen sich das Pulver vor der Inhalation in Kapseln befindet. In diesen Geräten werden die Kapseln in der Regel in irgendeiner Weise geöffnet, um das Pulver für die Zerstäubung zur Verfügung zu stellen: In manchen Geräten werden die Kapseln beispielsweise mit Messerklingen aufgeschnitten, in anderen wird ihr Inneres über Hohlnadeln an Luftwege im Inhalator angeschlossen. Bei einer Gruppe von Inhalatoren, die insbesondere den Hintergrund zu der hier vorliegenden Erfindung bilden, werden mit Hilfe von Nadelwerkzeugen Löcher in die Kapseln gestochen.
Die WO2004/082750 A1 zeigt ein Beispiel für einen solchen Inhalator, in dem eine Kapsel an ihren beiden Enden durch zwei entgegengesetzte Nadeln angestochen. Beim Inhalationsvorgang rotiert die Kapsel um ihre Querachse, wobei sie durch tangential einströmende Luft angetrieben wird. Partikel, die durch die Rotation der Kapsel aus ihrem Innern gefördert werden, bewegen sich dann durch den Luftstrom zum Mundstück.
Die US 5896855 zeigt einen Inhalator, in denen mehrere Kapseln in einem drehbaren Magazin bevorratet sind und die durch einen wahlweise motorgesteuerten Mechanismus einer Wirbelkammer zugeführt werden, wo das Pulver ebenfalls durch ein Rotieren der Kapsel um ihre Querachse aus Löchern an den Kapselenden ausgebracht wird. Im Magazin werden die Kapseln von Nadeln oder Stöpseln an beiden Enden festgehalten. Hierbei werden die Kapseln entweder vor Einsetzen in das Magazin an ihren Pol-Enden angestochen und diese Löcher werden durch die Stöpsel im Magazin bis zur Zuführung der jeweiligen Kapsel in die Wirbelkammer verschlossen; oder die Kapseln werden durch eben diese Nadeln beim Einsetzen der Kapseln ins Magazin angestochen und die Anstechnadeln verbleiben bis zur Zuführung der jeweiligen Kapsel in die Wirbelkammer abdichtend in den Löchern.

Die WO04/052435 A1 zeigt verschiedene Kapsel-basierte Pulverinhalatoren, in denen die Zerstäubung unter der Nutzung des so genannten Bernoulli-Effekts stattfindet. Ein gezeigter Inhalator weist ein Mundstück auf, das ähnlich wie eine Kappe ausgebildet ist und auf ein Unterteil aufgesetzt wird, das eine Kapselkammer beinhaltet. Am Gehäuseunterteil ist eine Schneidevorrichtung zum Öffnen der Kapseln vorgesehen. Zum Austausch der verbrauchten Kapseln gegen neue wird das Mundstück hochgeklappt oder eine Steckverbindung gelöst, die sich zwischen Mundstück und Gehäuseunterteil bzw. zwischen Mundstück und einer ins Gehäuseunterteil eingesetzten und mit der Kapselkammer verbundenen Platte befindet. Ein anderer gezeigter Inhalator weist ein drehbar gelagertes, austauschbares oder nachfüllbares Revolvermagazin mit mehreren, je mit einer Kapsel bestückten Kammern auf.

Solche den Bernoulli-Effekt nutzenden Pulverinhalatoren bilden den Ausgangspunkt für die hier vorgestellte Erfindung und die im Folgenden beschriebene Funktionsweise gilt auch für die Inhalatoren, die Gegenstand der vorliegenden Erfindungen sind.
In den hier betrachteten Inhalatoren ist der auszubringende Wirkstoff in einer im Wesentlichen zylindrischen Kapsel gelagert und diese Kapsel wird in die Inhalationskammer eines Inhalators eingesetzt. Die Kapselkammer ist dabei an die Größe der Kapsel insofern angepasst, dass sie ebenfalls im Wesentlichen zylindrisch ausgebildet ist, wobei ihre Länge und ihr Durchmesser jeweils etwas größer als die entsprechende Maße der Kapsel sind. Dadurch hat eine in die Kapselkammer eingesetzte Kapsel genug Spielraum, um sowohl in axialer als auch in radialer Richtung Vibrationsbewegungen ausführen zu können, dabei aber im wesentlichen entlang der Kammerachse ausgerichtet bleibt. Die Kapselkammer weist im Bereich eines der beiden Enden einen Lufteinlass und im Bereich des anderen Endes eine Luftauslassöffnung auf. Der Luftauslass ist an einen Inhalationskanal angeschlossen, der zum Mundstück des Inhalators führt. In der Regel sind Kapselkammer, Luftauslass, Inhalationskanal und Öffnung im Mundstück entlang einer gemeinsamen Achse angeordnet.
Zum Ausbringen des Inhalts der Kapsel wird die Kapsel zunächst an üblicherweise zwei Stellen längsseits am Mantel geöffnet. In der Regel befinden sich die Öffnungen in der Nähe der beiden längsseitigen Enden der Kapsel. Wird nun in der Kapselkammer ein Luftstrom vom Lufteinlass zum Luftauslass erzeugt, führt dieser entlang der Längsachse der Kapsel und bewirkt dabei zweierlei: Zum einen vibriert die Kapsel, wobei ihre Vorzugsbewegungsrichtung bedingt durch den Luftstrom entlang der Längsachse verläuft. Zum anderen erzeugt die an den beiden Kapselöffnungen entlang strömende Luft gegenüber dem Kapselinneren einen Unterdruck, so dass das in der Kapsel befindliche Pulver vom Luftstrom herausgesaugt und dabei zerstäubt wird.

Der vorliegenden Erfindung liegt die Aufgabe zugrunde, ein gegenüber dem Stand der Technik verbessertes System aus Kapsel und Inhalator anzugeben, bei dem die Reproduzierbarkeit der Zerstäubung, insbesondere die Pulverausbringung aus der Kapsel verbessert wird. Vorzugsweise soll ein System angegeben werden, bei dem Unregelmäßigkeiten beim Öffnen der Kapseln reduziert oder minimiert werden.
Besonders bevorzugt soll hierbei ein System bzw. ein Inhalator angegeben werden, das bzw. der sich für die einmalige Verwendung bzw. für die Verwendung als Wegwerfsystem bzw. Wegwerfprodukt eignet.

Die genannte Aufgabe wird erfindungsgemäß gelöst durch ein aus einem Inhalator und einer Kapsel bestehendes System gemäß Patentanspruch 1 und ein Verfahren zur Montage eines solchen Systems gemäß Patentanspruch 13.

Ein Merkmal der des erfindungsgemäßen Systems, das aus einer Kapsel und einem Inhalator gebildet wird, ist, dass der Inhalator eine Kapselkammer aufweist und die Kapsel vor der Anwendung des Inhalators in einer zum System oder zum Inhalator gehörenden Kapselaufnahme bevorratet ist. Die Kapsel dient der Verwendung als Reservoir für eine pharmazeutische Zubereitung bzw. medizinische Formulierung und umfasst zwei einseitig offene Kapselelemente, nämlich einen Kapselkörper und eine Kapselkappe, die zur Bildung eines Hohlraums teleskopartig über ihre Öffnungen ineinander gesteckt werden können. Kapselkörper und Kapselkappe sind dabei dadurch gekennzeichnet, dass mindestens einer von beiden, vorzugsweise beide, zusätzlich zur einseitigen Öffnung mindestens je ein vorgefertigtes Loch aufweisen. Die Kapselaufnahme dichtet mindestens ein Loch und/oder alle Löcher ab, das bzw. die nach dem Ineinanderstecken der Kapselelemente in den Hohlraum der Kapsel führt bzw. führen. Die Kapselaufnahme ist dabei zumindestens teilweise in der Kapselkammer angeordnet und derart aus der Kapselkammer entfernbar, dass die Kapsel bei Entfernung der Kapselaufnahme in der Kapselkammer zurückbleibt

Ein weiteres Merkmal der vorliegenden Erfindung ist, dass eine mit einer pharmazeutischen Zubereitung gefüllte Kapsel, die eine Kapselkappe und/oder einen Kapselkörper mit vorgefertigten Löchern aufweist, in einem Inhalator verwendet wird, wobei die vorgefertigten Löcher zum Zeitpunkt des Einsetzens der Kapsel in eine Kapselkammer des Inhalators verdeckt sind und durch Betätigung eines Zugelements im Inhalator freigelegt werden.

Dies ermöglicht die Bereitstellung eines kapselbasierten, vorzugsweise den Bernoulli-Effekt zur Zerstäubung nutzenden, Inhalators, in dem keine Anstechvorrichtungen zur Lochung der Kapsel bereit gestellt werden müssen. Das Freilegen von vorgefertigten Löchern bietet gegenüber der Verwendung von Anstechvorrichtungen im Inhalator verschiedene Vorteile: Die vorgefertigten Löcher sind bedingt durch ihren Fertigungsprozess, z.B. im Kunststoff-Spritzguss, in Größe und Form von Kapsel zu Kapsel sehr gut reproduzierbar während durch Anstechen erzeugte Löcher in Abhängigkeit von Kapselwerkstoff, Kapselgröße und Material so wie Anstechposition und Geometrie der Nadeln individuell variieren und/oder zu unregelmäßigen Lochgeometrien führen können. Des Weiteren ist beim Anstechen von Kapseln eine gewisse Rückformung der Kapseloberfläche im Bereich der Einstechstelle möglich. Die hier vorgestellten, vorgefertigten Löcher hingegen haben nach ihrer Freilegung eine stabile Form und keine Vorsprünge im Kapselmaterial. Solche Vorsprünge im Kapselmaterial bilden sich z.B. beim Eindrücken der Kapselwand durch eine Anstechvorrichtung und können möglicherweise zur Anlagerung von insbesondere pulverförmiger pharmazeutischer Zubereitung und somit bei Zerstäubung zu einer leicht verminderten Ausbringung der Zubereitung aus der Kapsel führen. Aufgrund der nun durch die Vorfertigung der Löcher möglichen hohen Präzisierung von der Größe und Form der Kapsellöcher sind sowohl die Menge als auch insbesondere die Verteilung des Pulvers reproduzierbarer, das aus Kapseln im Luftstrom in der Kapselkammer eines Inhalators ausgebracht wird. Die Zerstäubung der pharmazeutischen Zubereitung selbst wird dadurch reproduzierbarer, insbesondere im Vergleich zu Inhalatoren mit Kapselanstechvorrichtungen.

Ein weiteres Merkmal der vorliegenden Erfindung ist, dass der Inhalator zur Verwendung mit Kapseln mit vorgefertigten Löchern ein Zugelement aufweist und für die Betätigung des Zugelements Kanaläle bzw. Öffnungen benutzt werden, die bereits aufgrund des Funktionsprinzips des Inhalators notwendig sind. Das heißt insbesondere, dass das Zugelement in einem Luftkanal des Inhalators angeordnet ist. Für das Öffnen der Kapsel müssen somit an der Kapselkammer keine zusätzlichen Öffnungen ausgebildet werden, die das Strömungsverhalten bei der Zerstäubung nachteilig beeinflussen können. Es kommt nicht zur Bildung von unerwünschter Seitenluft bzw. Falschluft neben den für die Zerstäubung benötigten Luftkanälen. Das System ist hinsichtlich des Strömungswiderstandes stabil und reproduzierbar.

Vergleichsweise müsste bei der Verwendung einer Anstechvorrichtung das zugehörige Anstechelement durch eine oder mehrere Öffnungen in die Kapselkammer eingeführt werden, was während der Inhalation zu zusätzlichen unerwünschten Luftströmungen durch diese Öffnungen führen kann. Solche Öffnungen zusätzlich zu Lufteinlass und Luftauslass der Kapselkammer werden beim erfindungsgemäßen Inhalator nicht benötigt, sofern sie nicht ihrerseits auf eine Verbesserung der Luftströmung in der Kapselkammer ausgerichtet sind.
Die Verwendung eines Zugelements zum Freilegen von vorgefertigten Löchern hat im Vergleich zur Verwendung von Anstechelementen einen weiteren Vorteil: bei der Verwendung von Anstechelementen kann es bedingt durch den für den Zerstäubungsprozess notwendigen Bewegungsspielraum der Kapsel in der Kapselkammer zu Schwankungen bei der Position der Kapsel zu den Anstechspitzen kommen, wenn die Kapsel in der Kapselkammer angestochen wird. Dies führt im Vergleich unter mehreren gleichartigen Kapsel-Inhalator-Systemen zu Schwankungen bezüglich der exakten Position der Löcher in der jeweiligen Kapsel.
Durch die Verwendung eines Zug-Mechanismus zur Freilegung von vorgefertigten Löchern an Kapseln werden alle aus der Verwendung von Anstechvorrichtungen resultierenden Schwankungen in der Form, Größe und Position der Löcher und die sich daraus ergebenden Schwankungen in der Ausbringung der pharmazeutischen Zubereitung vermieden. Die Zerstäubung der pharmazeutischen Zubereitung ist nun unabhängig von dem Mechanismus zum Öffnen der Löcher in der Kapsel. Dies bedeutet auch, dass bei Verwendung von brüchigem Kapselmaterial das Risiko einer Splitterung, wie sie insbesondere bei Anstechvorgängen auftreten könnte, minimiert ist. Auf diese Weise erweitert sich die Gruppe möglicher Kapselmaterialien für die Verwendung in Inhalatoren.

Die Verwendung eines Zug-Mechanismus zum Freilegen vorgefertigter Löcher hat des Weiteren je nach Ausgestaltung des Zugelements den Vorteil, dass der gleiche Mechanismus für die Freilegung unterschiedlichster Lochanordnungen an Kapseln benutzt werden kann. Z.B. können sich bei zylindrischen Anordnungen von Kapsel, Kapselkammer und Zugelement mehrere Löcher an der Kapsel an - bezogen auf ihren Umfang - unterschiedlichen Stellen befinden. Größe, Form, Position, Anzahl der Löcher in einer Kapsel sind bei diesem Konzept sehr variabel, insbesondere bei einer Fertigung der Kapselteile aus Spritzgussprozessen. An dieser Stelle können die Ergebnisse von auf die Zerstäubung von bestimmten pharmazeutischen Zubereitungen bzw. bestimmten Pulvern abgestimmten Strömungssimulationen benutzt werden, um optimale Lochstrukturen für Kapseln für diese bestimmte Zubereitung bzw. dieses bestimmte Pulver zu verwenden.

Ein weiteres Merkmal der Erfindung, das auch unabhängig von den anderen Merkmal der Erfindung allgemein für Inhalatoren anwendbar ist, ist, dass der Inhalator einen Körper (100) aufweist, der eine Kammer wie insbesondere eine Vibrationskammer oder bevorzugt eine Kapselkammer (74) zur Aufnahme einer vorzugsweise Pulver-gefüllten Kapsel (71) bildet, und das Material dieses Körpers (100) im Wesentlichen aus einer Tiefziehfolie oder einer Blisterfolie besteht. Die Verwendung solcher aus dem Bereich der Verpackung von medizinischen Tabletten hinsichtlich ihrer Kompatibilitäten mit medizinischen Wirkstoffen gut erforschten Materialien ermöglicht eine sehr kostengünstige Herstellung von Inhalatoren, die sonst mit größerem Materialaufwand z.B. in Kunststoff-Spritzgussverfahren gefertigt werden.
Bevorzugt wird der Körper aus zwei Hälften oder Teilen gebildet, die durch Siegeln, Laminieren, Kleben oder Schweißen miteinander verbunden werden. Vorzugsweise wird das Reservoir, das eine zu zerstäubende bzw. auszubringende medizinische Zubereitung enthält, oder speziell wie hier bevorzugt eine Kapselaufnahme mit einer Pulver-gefüllten Kapsel in die Kammer eingelegt, bevor die beiden Hälften oder Teile des Körpers miteinander verbunden werden. Dieses aus nur wenigen Arbeitsschritten bestehende Verfahren ermöglicht einen unkomplizierten und somit schnellen und kostengünstigen Montageprozess. Durch die insgesamt im Vergleich zur Produktion handelsüblicher Inhalatoren gesenkten Herstellungskosten eignen sich die derart aus Tiefziehteilen hergestellten Inhalatoren sehr gut für die Verwendung als Einweg-Inhalatoren, d.h. als Produkte für den Einmaligen Gebrauch bzw. als sogenannte Wegwerf-Artikel.
Darüber hinaus ist das Prinzip der kostengünstige Montage von zwei Inhalator-Teilen, die eine innere Kammer vorzugsweise für ein eingeschlossenes Objekt bilden, auch auf Komponenten übertragbar, die aus Kunststoff-Spritzguss (statt aus Tiefziehteilen) gefertigt wurden: Zwei solche Komponenten können entlang ihrer Verbindungsflächen z.B. durch Ultraschallschweißen miteinander verbunden werden.

Vorteilhafte Weiterbildungen werden im Folgenden und im Detail anhand der Figuren beschrieben.

In einer Ausführungsform, wird das mindestens eine in der Kapsel vorgefertigte Loch durch ein Zugelement verschlossen, wobei das Zugelement eine Kapselaufnahme bildet oder eine Kapselaufnahme Teil des Zugelements ist. Diese Kapselaufnahme hat im Wesentlichen die Gestalt einer zylindrischen Röhre, die so bemessen ist, dass sie den zylindrischen Teil der Kapsel passgenau umschließt. Das mindestens eine vorgefertigte Loch in der Kapsel befindet sich in dem zylindrischen Mantelbereich der Kapsel und wird somit bei der Bevorratung oder Lagerung der Kapsel im Inhalator durch die Wand der Kapselaufnahme verschlossen. Vorzugsweise weist die so bevorratete und mit Pulver gefüllte Kapsel zwei Löcher oben und unten auf, d.h. je ein Loch am Anfang und eins am Ende des zylindrischen Mantelbereichs. Vorzugsweise besteht die Kapsel aus einem Kapselkörper und einer Kapselkappe, die beide mindestens ein vorgefertigtes Loch haben, wobei nach Zusammenschieben von Kapselkörper und Kapselkappe ineinander die Löcher frei bleiben, d.h. nicht vom jeweils anderen Kapselelement abgedeckt werden. Alternativ können die Löcher auch nach Befüllung der Kapsel und nach Zusammenführung von Kapselkappe und Kapselkörper außerhalb des Inhalators vorgefertigt werden. In den Kapseln können beispielsweise Pulvermengen zwischen 0,1 Milligramm und 100 Milligramm eines reinen Wirkstoffs oder einer Wirkstoffmischung, zur späteren Ausbringung bevorratet sein.

In einer bevorzugten Ausgestaltung des erfindungsgemäßen Systems Systems aus Inhalator und Kapsel, insbesondere im Falle eines Systems zur einmaligen Anwendungen (Einwegsystem), weist dieses einen Zugmechanismus zum Freilegen der vorgefertigten Kapsellöcher auf. Der Zugmechanismus ist vorzugsweise derart gestaltet, dass sich die Kapsel in einer Kapselaufnahme befindet und sich die Kapselaufnahme in einem für Transport und Lagerung geeigneten Zustands des Systems in der Kapselkammer befindet. Um das System anwendungsbereit zu machen wird die Kapselaufnahme aus der Kapselkammer, vorzugsweise auch komplett aus dem Inhalator, herausgezogen. Dabei ist eine Vorrichtung vorgesehen, die verhindert, dass die Kapsel mit der Kapselaufnahme aus der Kapselkammer herausgezogen wird und/oder die Kapsel aus der Kapselaufnahme beim Herausziehen der Kapselaufnahme löst.

Vorzugsweise wird die Kapselaufnahme durch ein Mundrohr am Inhalator herausgezogen, das Mundrohr bildet hierbei den Luftauslass aus der Kapselkammer in Richtung mundseitigem Ende des Inhalators. Diese Vorrichtung zur Rückhaltung der Kapsel in der Kapselkammer beinhaltet vorzugsweise einen Stab oder Steg, der gleichzeitig die obere Begrenzung der Kapselkammer bildet. Dieser Stab oder Steg durchdringt vorzugsweise im Lagerzustand des Systems die Kapselaufnahme auf der einem Lufteinlass der Kapselkammer entgegengesetzten Seite. Die Kapselaufnahme weist vorzugsweise schlitzförmig ausgebildete Aussparungen auf, aufgrund derer die Kapselaufnahme beim Herausziehen aus dem Inhalator an dem Steg vorbeigleiten und sich somit von ihm trennen kann. Vorzugsweise weist das die Kapselaufnahme bildende Bauteil im Transportzustand des Systems einen aus dem Inhalator herausragenden Bereich auf, an dem eine Grifffläche ausgebildet ist, an welcher der Anwender anfassen kann, um die Kapselaufnahme aus dem Inhalator herausziehen kann. Besonders bevorzugt ist darüber hinaus das die Kapselaufnahme bildende Bauteil als Kappe gestaltet, die im Transportzustand das mundseitige Ende des Inhalators abdeckt.

In Abhängigkeit vom Abdichtungskonzept der vorgefertigten Löcher in der Kapsel wird die Kapselaufnahme bzw. das Zugelement ausgelegt, mit dessen Hilfe die Löcher abgedichtet bzw. freigelegt werden. In einer bevorzugten Ausführungsform wird die Kapselaufnahme vom Zugelement gebildet. Vorzugsweise werden Kapselaufnahme und Zugelement als ein oder mehrere Tiefziehteile aus einer Blisterfolie gefertigt. Vorzugsweise durch einen Siegelprozess am Zugelement werden die Kapseln in der Kapselaufnahme im Bereich der vorgefertigten Löcher verschlossen. Vorzugsweise ist die Kapselaufnahme dabei derart flexible ausgelegt, dass die Kapsel zerstörungsfrei durch Druck entlang der Längsachse des Zugelements aus der Kapselaufnahme gelöst werden kann; z.B. wie eben beschrieben, dadurch dass bei Betätigung des Zugmechanismus ein Stab oder anderes Hindernis die Kapsel zurückhält während die Kapselaufnahme aufgrund eine geeigneten Schlitzes an dem Stab oder Hindernis vorbei gleiten kann.

Ein weiteres Merkmal der Erfindung ist, dass im Fall von Systemen für einmalige Anwendung die Bauteile, die am jeweiligen Zugmechanismus beteiligt sind, Vorrichtungen aufweisen, die bewirken, dass die jeweilige Zug-Bewegung nicht zerstörungsfrei rückgängig gemacht werden kann. Insbesondere weist hierzu die Kapselaufnahme Federarme oder sonstige Elemente auf, die im Fall der herausgezogenen Kapselaufnahme ein zerstörungsfreies Wiedereinsetzen der Kapselaufnahme in die Kapselkammer verhindert.

Die beschriebenen Systeme für einmalige Anwendung können alternativ auch als Systeme mit zwei Kapseln ausgelegt werden. Der Inhalator weist bei solchen Ausführungsformen für die zwei Kapseln auch zwei zugehörige zwei Kapselkammern und zwei Kapselaufnahmen aufweist. Je nach Einsatzzweck eines solchen Systems mit zwei Kapseln, sind die beiden Kapseln optional mit verschiedenen Formulierungen und/oder verschiedenen Formulierungsmengen befüllt. Ein solches System aus Inhalator und zwei Kapseln kann beispielsweise in Therapien verwendet werden, in denen zwei verschiedene medizinische Formulierungen gleichzeitig einem Patienten verabreicht werden sollen. Bei verschieden befüllten Kapseln kann es zweckdienlich sein, die Größe der Kapseln auf die jeweilige Füllmenge anzupassen. In einer solchen sehr speziellen Ausführungsform sind dann auch die jeweiligen Kapselkammern und Kapselaufnahmen hinsichtlich ihrer Größen an ihre jeweiligen Kapseln angepasst. Unabhängig davon, ob es sich um eine Ausführungsform mit zwei gleichen oder zwei verschiedenen Kapseln handelt, sind bei einem solchen System mit zwei Kapseln der Zugmechanismus am Inhalator so gestaltet, dass mit einer Bewegung gleichzeitig die Löcher an beiden Kapseln freigelegt werden. Weist das System beispielsweise Kapselaufnahmen, die sich in Mundrohren des Inhalators erstrecken, und eine Kappe mit einer Grifffläche oder einer Lasche zum Ziehen auf, so werden beim Ziehen an der Kappe beide vorzugsweise im Kappenbereich miteinander verbundene Kapselaufnahmen gleichzeitig aus dem Inhalator gezogen.

In einer anderen Ausgestaltung eines erfindungsgemäßen Systems wird das System aus einem Inhalator und einer zusammengesetzten Kapsel mit mindestens einem vorgefertigtes Loch gebildet, wobei sich die Kapsel in einem Transportzustand des Systems im Inhalator befindet und dabei zumindest teilweise derart von einer vorzugsweise dehnbaren und/oder biegsamen Folie umschlossen ist, dass die Folie das mindestens eine vorgefertigte Loch und/oder alle Löcher der Kapsel im Transportzustand des Systems verschließt. Dabei ist die Folie vorzugsweise mit einem Zugband oder einem sonstigen Griffelement verbunden und/oder ragt zum Teil an einem Ende der Kapsel über die Kapsel hinaus (die Folie mit Zugband oder die rausragende Folie selbst ist in diesen Ausgestaltungen also das "Zugelement"). Der Inhalator weist eine Öffnung auf, durch welche die Folie an ihrem überstehenden Teil und/oder an ihrem Zugband aus dem Inhalator herausgezogen werden kann, wobei die vorgefertigten Löcher an der Kapsel frei gelegt werden.

Aufgrund der geringen Komplexität eines auf einem Zugmechanismus basierenden Systems und der damit verbundenen geringen Fertigungskosten, eignet sich ein solches System aus Kapsel mit vorgefertigten, vorerst verdeckten Löchern und in eine Kapselkammer eingesetzte Kapselaufnahme bzw. eingesetztes Zugelement auch für die Fertigung von Einzeldosis-Inhaltoren (Wegwerfartikel). Insbesondere bei Anwendungen, bei denen klebrige pulverförmige pharmazeutischen Zubereitungen zur Inhaltion bereit gestellt werden sollen, bilden sich schnell Ablagerungen in Kapselkammer, Luftauslass und Mundstück, so dass ein häufiger Austausch eines solchen Systems gewünscht ist, wie er durch ein Einweg-Produkt gegeben ist.

In einer Ausgestaltung des erfindungsgemäßen Systems aus Inhalator und Kapsel kann die zusammengesetzte Kapsel in zwei verschiedenen Zuständen vorliegen: Im ersten Zustand ist das mindestens eine vorgefertigte Loch geschlossen, in der zweiten liegt es frei.

Somit weisen beide Kapselelemente vorgefertigte Löcher und beim teleskopartigen Ineinanderstecken zwei Einsteckstellungen zueinander auf (Diese beiden Einsteckstellungen entsprechen in dieser Ausführung dem so genannten ersten und zweiten Zustand der Kapsel): Eine erste Einsteckstellung, in welcher beide Elemente so ineinander gesteckt sind, dass die vorgefertigten Löcher verdeckt sind und der Hohlraum der Kapsel insgesamt geschlossen ist, und eine zweite Einsteckstellung, in der sich die vorgefertigten Löcher in Kapselkörper und Kapselkappe so überdecken, dass die gesamte Kapsel an der Stelle der Überdeckung beider Löcher ein Loch aufweist.
Vorzugsweise sind beide Kapselelemente becherartig geformt: Der von ihnen gebildete, einseitig offene Hohlraum wird seitlich durch einen umlaufenden Kapselmantel und gegenüber der offenen Seite durch ein geschlossenes Ende begrenzt. Vorzugsweise bildet der Kapselmantel eine zylindrische oder elliptisch umlaufende Wand, so dass in der zusammengeschobenen Kapsel innen keine Ecken gebildet werden, in denen sich insbesondere pulverförmige pharmazeutische Zubereitung ansammeln und dadurch bei einer späteren Ausbringung des Pulvers aus der Kapsel zurück bleiben könnte. Aus gleichem Grund haben die Unterseiten von Kapselkörper und Kapselkappe und somit beide Enden der beim Ineinanderstecken resultierenden Kapsel eine konvexe, insbesondere im Wesentlichen halbkugelartige oder ellipsoidale Form.
Die vorgefertigten Löcher in Kapselkappe und Kapselkörper befinden sich vorzugsweise in ihrem jeweiligen Mantelbereich, so dass beim Ineinanderstecken jeweils der Mantel des anderen Kapselelements das jeweilige Loch abdeckt, bis die Einsteckstellung erreicht ist, in der beide Löcher zur Überdeckung gebracht werden.
Darunter, dass ein Loch "vorgefertigt" ist, ist zu verstehen, dass das Loch in dem jeweiligen Kapselelement bereits im werksseitigen Herstellprozess der Kapseln entsteht. Das Loch im jeweiligen Kapselelement ist hierbei bereits vorhanden, bevor die einzelnen Kapsel fertig zusammengesetzt werden. Insbesondere ist das Loch bzw. sind die Löcher in den Kapselelementen bereits vorhanden, bevor die Kapsel als ganzes in einen Inhalator eingesetzt wird.

Bevorzugt sind Kapselkappe und Kapselkörper auf jeweils den Seiten, die im ineinandergesteckten Zustand aneinander liegen, im Kapselmantelbereich strukturiert. Diese Strukturierung ist vorzugsweise so ausgelegt, dass sie unterschiedliche Funktionen erfüllt. Zum einen weisen die Strukturen von Kapselkappe und Kapselkörper wechselseitige Rastelemente auf. Bevorzugt bewirkt diese Struktur beim Ineinanderstecken der Kapselelemente, dass die Kapselkappe und der Kapselkörper in Relation zu einander in mindestens zwei Stellungen, insbesondere den genannten Einsteckstellungen, verrasten. Durch eine Verrastung in der ersten Einsteckstellung wird sichergestellt, dass zum einen die Löcher in den Kapseln nicht zufällig z.B. durch Erschütterungen beim Transport vorzeitig freigelegt werden und ein definierter Vorgang wie das Zusammenschieben beider Elemente unter Ausübung eines definierten Drucks notwendig ist, um die Löcher freizulegen. Des Weiteren sind die miteinander wechselwirkenden Rastelemente von Kapselkappe und Kapselkörper vorzugsweise so gestaltet, dass sich die beiden Kapselelemente nach dem Ineinanderstecken bis zur ersten Einsteckstellung nicht mehr zerstörungsfrei von einander trennen lassen. Dadurch wird verhindert, dass sich die Kapsel zufällig öffnet. Vorzugsweise sind im Kontaktbereich zwischen Kapselkappe und Kapselkörper zusätzlich Strukturelemente vorhanden, die als Führung beim Ineinanderstecken der beiden Kapselelemente dienen. Diese Strukturelemente an den zueinander gerichteten Mantelbereichen von Kapselkappe und Kapselkörper bewirken, dass die beiden Kapselelemente nur in definierten Ausrichtungen ineinander gesteckt werden können. Die "definierte Ausrichtung" bezieht sich auf die Drehung der Kapselelemente um die Längsachse der Kapsel, es ist also eine azimutale Ausrichtung. Vorzugsweise haben diese Strukturelemente die Gestalt mindestens einer Nut in der äußeren / inneren Mantelfläche von Kapselkappe / Kapselkörper und respektive die Gestalt mindestens einer Führungsschiene in der inneren / äußeren Mantelfläche von Kapselkörper / Kapselkappe. Auf diese Weise wird sichergestellt, dass beim Ineinanderstecken der Kapselelemente die vorgefertigten Löcher in Kapselkappe und Kapselkörper sicher zur Überdeckung gebracht werden können. Bevorzugt werden hierbei geradlinige Führungen, insbesondere solche parallel zur Hauptachse der Kapsel. Varianten in denen die Strukturelemente die Bewegung der Kapselelemente beim Ineinanderstecken gekurvt führen sind ebenfalls möglich. Dies würde beispielsweise durch eine schraubenförmige Führungsnut oder Führungsschiene bewirkt werden.

Ein weiteres - sowohl unabhängig als auch in Kombination mit den oben genannten Aspekten umsetzbares - Merkmal der vorliegenden Erfindung ist, dass die Kapsel statt eines zylindrischen Querschnitts einen elliptischen Querschnitt aufweist. Vorzugsweise handelt es sich bei der Ellipse um so eine, die nur leicht von der Form eines Kreises abweicht (Das Verhältnis von Längs-zu-Querachse der Ellipse sollte kleiner als 75%, vorzugsweise zwischen 90% und 85% sein). Ein solcher elliptischer Querschnitt erzwingt eine definierte azimutale Ausrichtung der beiden Kapselelemente beim Ineinanderstecken und/ oder eine definierte Orientierung beim Einsetzen einer resultierenden Kapsel in eine Kapselaufnahme.

Ein weiteres Merkmal der vorliegenden Erfindung ist, dass das Loch im äußeren Kapselelement vorzugsweise als Langloch, elliptisch oder etwas größer als das zugehörige Loch im inneren Kapselement gestaltet ist. Bei Ausführungen des äußeren Lochs als Langloch oder als Ellipse ist vorzugsweise der schmale Durchmesser des Lochs mindestens so groß wie der Durchmesser des Lochs im inneren Kapselelement. Das äußere Kapselelement ist jenes, das von Kapselkörper und Kapselkappe im ineinandergesteckten Zustand im Bereich der aneinanderliegenden Kapselmäntel die Außenwand der Kapsel bildet. Durch diese Vergrößerung des äußeren Lochs gegenüber dem inneren werden die Toleranzen hinsichtlich der Genauigkeit des Ineinanderstecks der Kapselelemente aufgeweitet. Somit wird sichergestellt, dass auch bei unpräzisem Aufeineinanderschieben die volle Kapselöffnung zur Verfügung steht.

Die einzelnen Merkmale der vorliegenden Erfindung können unabhängig voneinander genutzt oder miteinander kombiniert werden.
Weitere Vorteile, Merkmale, Eigenschaften und Aspekte der vorliegenden Erfindung ergeben sich aus den Ansprüchen und der folgenden Beschreibung bevorzugter Ausführungsformen anhand der Zeichnungen. Die Zeichnungen zeigen in:
Fig. 1 eine schematische Darstellung einer speziellen Kapsel in unterschiedlichen Zuständen: a) Kapselkappe und Kapselkörper vor dem Ineinanderstecken, b) Kapselkappe und Kapselkörper in einer ersten Einsteckstellung und c) Kapselkappe und Kapselkörper in einer zweiten Einsteckstellung;
Fig. 2 eine schematische Darstellung einer zweiten Ausführungsform der Kapsel in unterschiedlichen Zuständen: a) Kapselkappe und Kapselkörper vor dem Ineinanderstecken und b) Kapselkappe und Kapselkörper in einer zweiten Einsteckstellung;
Fig. 3 eine schematische Darstellung einer dritten Ausführungsform der Kapsel, wobei Kapselkappe und Kapselkörper vor dem Ineinanderstecken gezeigt werden;
Fig. 4 eine schematischen Querschnitt einer vierten Ausführungsform der Kapsel, wobei in der ersten Bildhälfte Kapselkappe und Kapselkörper vor dem Ineinanderstecken und in der zweiten Bildhälfte Kapselkappe und Kapselkörper nach dem Ineinanderstecken gezeigt werden.
Fig. 5 eine schematischen Querschnitt einer fünften Ausführungsform der Kapsel, wobei in der ersten Bildhälfte Kapselkappe und Kapselkörper vor dem Ineinanderstecken und in der zweiten Bildhälfte Kapselkappe und Kapselkörper nach dem Ineinanderstecken gezeigt werden.
Fig. 6 eine schematische Darstellung einer sechsten Ausführungsform der erfindungsgemäßen Kapsel in unterschiedlichen Zuständen: Fig. 6a zeigt Kapselkappe, Kapselkörper und Ring vor dem Ineinanderstecken, Fig. 6b zeigt Kapselkappe getrennt von den zusammengeschobenen Bauteilen Ring und Kapselkörper, Fig. 6c zeigt Kapselkappe und Kapselkörper in einer zweiten Einsteckstellung.
Fig. 7 eine schematische Darstellung einer siebten und einer achten Ausführungsform einer Kapsel: Fig. 7a zeigt die geschlossene Kapsel mit Füllung, Fig. 7b zeigt die Position von Löchern der Kapsel gemäß der siebten und Fig. 7c der achten Ausführungsform.
Fig. 8 eine schematische Darstellung einer neunten Ausführungsform der erfindungsgemäßen Kapsel mit zugehöriger Funktionsweise eines Inhalators mit Schiebemechanismus: Fig. 8a zeigt die Kapsel mit vorgefertigten Löchern, Fig. 8b die Kapsel bevorratet in einem Inhalator und Fig. 8c die Kapsel nach Verschiebung aus ihrem Lagerzustand in die Kapselkammer des Inhalators.

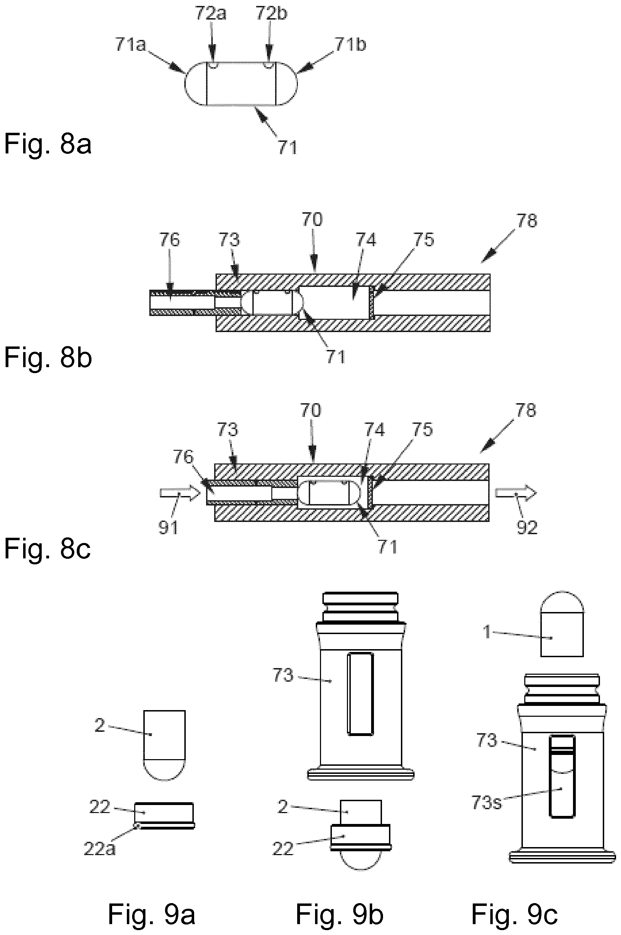

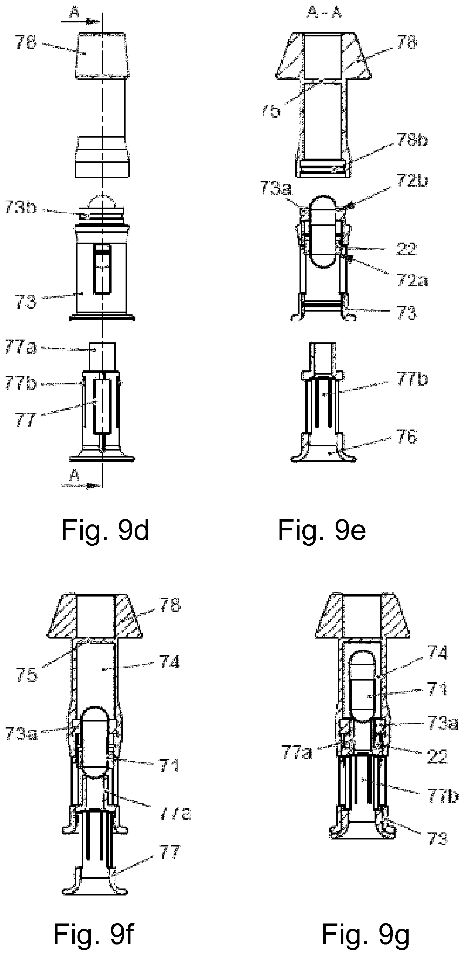

Fig. 9 eine schematische Darstellung der Funktionsweise einer zweiten Ausführungsform eines Inhalators mit Schiebemechanismus und des Aufbaus des Systems aus Kapsel (ähnlich der sechsten oder neunten Ausführungsform) und Inhalator: Fig. 9a zeigt ein Kapselelement mit zugehöriger ringförmiger Halterung, Fig. 9b die Röhre des Inhalators, in welche Kapsel und Ring eingesetzt werden, Fig. 9c den Verschluss der Kapsel in der Röhre, Fig. 9d eine Explosionsdarstellung der Bauteile bzw. Bauteilgruppen des Inhalators, Fig. 9e die gleiche Explosionsdarstellung in schematischem Längsschnitt, Fig. 9f das fertig zusammen gesetzte System aus Kapsel und Inhalator im Transportzustand und Fig. 9g das System im anwendungsbereiten Zustand.

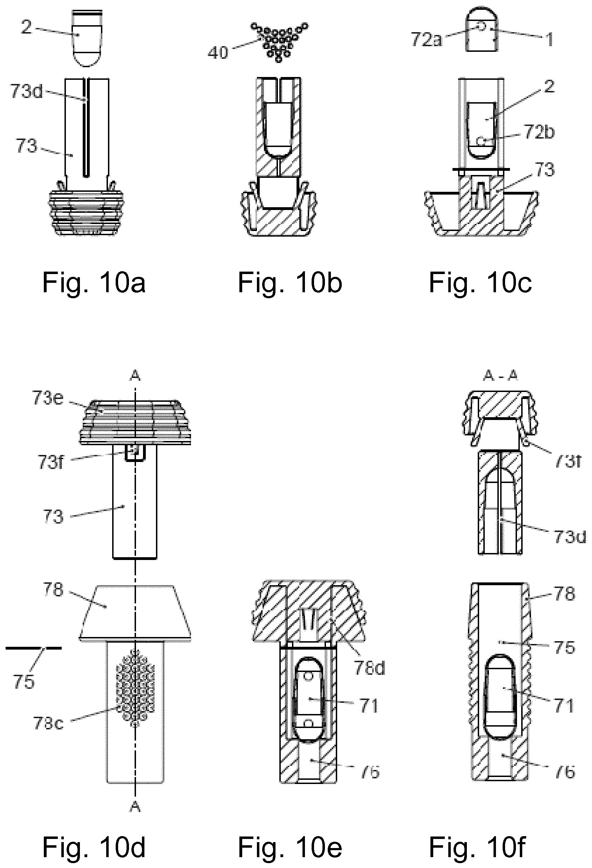

Fig. 10 eine schematische Darstellung der Funktionsweise einer ersten Ausführungsform eines erfindungsgemäßen Inhalators und des Aufbaus des zugehörigen erfindungsgemäßen Systems aus Kapsel (ähnlich der achten oder neunten Ausführungsform) und Inhalator: Fig. 10a zeigt den Einsatz eines Kapselelements in eine Röhre, Fig. 10b die Befüllung des Kapselelements mit Pulver im schematischen Längsschnitt durch die Röhre, Fig. 10c den Verschluss der Kapsel in der Röhre im schematischen, gegenüber Fig. 10b um 90° gedrehten Längsschnitt der Röhre, Fig. 10d eine Explosionsdarstellung der Bauteile bzw. Bauteilgruppen des Inhalators, Fig. 10e einen schematischem Längsschnitt des fertig zusammen gesetzten System aus Kapsel und Inhalator im Transportzustand und Fig. 10fb einen schematischen Längsschnitt des Systems aus Kapsel und Inhalator im Anwendungszustand.
Fig. 11 einen schematischen Längsschnitt eines Systems aus zwei Kapseln (ähnlich der achten oder neunten Ausführungsform) und einem Inhalator gemäß einer zweiten erfindungsgemäßen Ausführungsform.
Fig. 12 einen schematischen Längsschnitt eines Systems aus Kapsel und Inhalator gemäß einer dritten erfindungsgemäßen Ausführungsform; Fig. 12a einen Schnitt des fertig zusammen gesetzten System aus Kapsel und Inhalator im Transportzustand und Fig. 12b einen Schnitt des Systems aus Kapsel und Inhalator im Anwendungszustand.

Die hier betrachteten Ausführungsformen der Inhalatoren die zusammen mit einer mit einer Kapsel betrieben werden, basieren bevorzugt auf dem so genannten Bernoulli-Prinzip: Die Inhalatoren (z.B. zu sehen an Fig. 8c) weisen eine Kapselkammer (74) auf, deren Länge derart auf die Länge der Kapsel (71) angepasst ist, dass sich die Kapsel (71) gemäß dem Bernoulli-Effekt im Luftstrom bewegen und vibrieren kann. Die Kapselkammer (74) weist hierzu einen Einlass (76) für die Luft und einen Luftauslass auf. Die Begrenzung der Kapselkammer (74) in Richtung des Luftauslasses wird vorzugsweise durch einen Stab (75), Sieb und/oder durch ein aerodynamisch vorteilhaft geformtes Bauteil gebildet, das nur einen geringen Strömungswiderstand darstellt. An diese den Luftauslass beinhaltende Begrenzung der Kapselkammer (74) schließt sich ein Mundstück (78) an, an dem der nicht dargestellte Anwender inhaliert und dadurch den zur Zerstäubung des Kapselinhalts notwendigen Luftstrom erzeugt.

Im Folgenden werden verschiedene spezielle Kapseln zur Verwendung in Inhalatoren mit Bernoulli-Funktionsweise beschrieben.

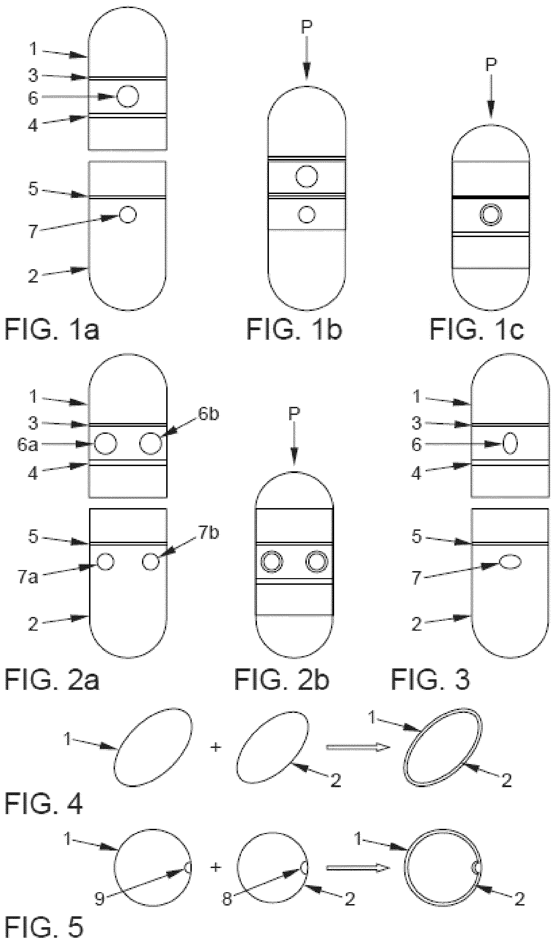

Figur 1 zeigt in schematischer Darstellung eine spezielle Kapsel bestehend aus Kapselkappe (1) und Kapselkörper (2), die beide eine becherartige Form haben und teleskopartig über ihre Öffnungen ineinander gesteckt werden können. Diese und alle nachfolgenden Darstellungen sind als Skizzen zu verstehen, in denen Wandstärken und ähnliche Details nicht in vollem Umfang dargestellt bzw. teilweise nicht unbedingt maßstabsgerecht zueinander abgebildet sind.
Die in dieser und den folgenden Figuren dargestellten Kapseln werden vorzugsweise mit einer pulverförmigen Arzneimittelzubereitung gefüllt. Bevorzugt haben die Kapselkappe (1) und der Kapselkörper (2) die Form eines auf einer Seite offenen Zylinders mit rundem Querschnitt und konvexer, nahezu halbkugelförmiger geschlossener anderer Seite. Kapselkappe (1) und Kapselkörper (2) bestehen beide vorzugsweise aus Polypropylen (PP) oder Polyethylen (PE), besonders bevorzugt aus high-density Polyethylen mit einer Dichte zwischen 950 und 1000 kg/m³. Alternativ sind auch Ausführungsformen möglich bei denen Kapselkappe (1) und Kapselkörper (2) aus unterschiedlichen Materialien sind, beispielsweise Kapselkörper aus PP oder PE und die Kapselkappe aus Gelatine. Die Kapselgrößen sind an die jeweiligen Inhalatoren bzw. die Dimensionen der darin enthaltenen Kapselkammern angepasst, in denen sie eingesetzt werden sollen. Typische Längen der zusammengefügten Kapseln sind beispielsweise 9 mm bis 22 mm bei äußeren Durchmessern von 4 mm bis 10 mm. Beispiele für die Kapseldimensionen sind der Offenbarung der WO2006/074982 A2 auf Seite 6 Zeile 6 bis 27 zu entnehmen.
Bezüglich der Materialauslegung der Kapsel, für die neben dem hier bevorzugten Polyethylen auch alle pharmazeutisch akzeptablen Kunststoffe in Frage kommen, wird in der Anmeldung WO2006/074982 A2 auf Seite 5 Zeile 6 bis 31 beschrieben Figur 1a zeigt die beiden getrennten Kapselelemente (1) und (2) mit den vorgefertigten Löchern (6) und (7) vor dem Ineinanderstecken. In der hier dargestellten Ausführungsform wird beim teleskopartigen Ineinanderschieben die Kapselkappe (1) auf den Kapselkörper (2) aufgesetzt. (Auch der umgekehrte Fall des Einschiebens der Kapselkappe (1) in den Kapselkörper (2) hinein ist denkbar; für diesen Fall müssten alle nachfolgenden Beziehungen bzgl. "innen" und "außen" gerade umgekehrt betrachtet werden). Für die hier gewählte Ausführungsform mit Aufsetzen der Kapselkappe (1) auf den Kapselkörper (2) ist der äußere Durchmesser der Kapselkappe (1) im Bereich ihrer Becheröffnung etwas größer als der Kapselkörper (2). Der äußere Durchmesser des Kapselkörpers (2) ist an dieser Stelle von vergleichbarer Größe wie der innere Durchmesser der Kapselkappe (1), wobei die Durchmesser von ihren Toleranzen her so aufeinander abgestimmt sind, dass die Kapselelemente beim Fügen ohne nennenswerte Spaltbildung im Bereich der Kapselmantelbereiche ineinander passen. In der gewählten Darstellung bildet somit nach Ineinanderstecken der Kapselelemente der Kapselkörper (2) die innere Wand der Kapsel im Bereich der zwei aufeinander liegenden Mantelbereiche der Kapselelemente.
Die Kapsel wird mit der vorzugsweise pulverförmigen Arzneimittelzubereitung gefüllt, z.B. durch Einfüllen der Zubereitung in den Kapselkörper. Nach der Füllung wird die Kapselkappe (1) bis zu einer ersten Einsteckstellung auf den Kapselkörper (2) aufgeschoben. Der mit "p" gekennzeichnete Pfeil in den Abbildungen kennzeichnet die Richtung, in der die Kapselelemente zusammengeschoben werden bzw. zusammengeschoben worden sind. Er soll des Weiteren den Druck symbolisieren, der für dieses Zusammenschieben angewendet werden muss.
In der ersten Einsteckstellung (siehe Fig. 1b) greifen Rastelemente am äußeren Mantelbereich des Kapselkörpers (2) und Rastelemente am inneren Mantelbereich der Kapselkappe (1) ineinander. In allen hier dargestellten Figuren sind diese Rastelemente in Form von ringförmig umlaufenden Vorsprüngen oder Wülsten und Nuten oder Sicken dargestellt. So hat der Kapselkörper (2) in Figur 1 beispielsweise eine nach außen gerichtete umlaufende Wulst (5), die in der ersten Einsteckstellung in eine erste ringförmig umlaufende Sicke (4) innen an der Kapselkappe (1) einrastet. Die gleiche Wirkung würde erzielt durch eine ringförmige Nut im äußeren Mantelbereich des Kapselkörpers (1), wobei in der Einsteckstellung ein ringförmig umlaufender Vorsprung am inneren Mantelbereich der Kapselkappe (1) in diese Nut einrastet. Die Rastelemente müssen jedoch nicht zwangsläufig ringförmig ausgebildet sein, sondern können auch durch eher punktförmige Erhebungen in Kapselkörper und passende Vertiefungen in Kapselkappe bzw. umgekehrt gebildet werden. In einer bevorzugten Ausführungsform weist der Kapselkörper mehrere punktförmige, ringförmig angeordnete Erhebungen auf, die in eine entsprechende, vorzugsweise ringförmig umlaufende, Nut außen an der Kapselkappe einrasten. Bezüglich der Auslegung einer solchen eher punktuell gebildeten Verrastung sei hiermit als Beispiel auf die Offenbarung der WO2006/074982 A2 auf Seite 7 Zeile 1 bis Seite 8 Zeile 32 verwiesen. In der ersten Einsteckstellung (siehe Figur 1b) überlappen sich die Mantelbereiche von Kapselkörper (2) und Kapselkappe (1) vorzugsweise so, dass der Mantel des Kapselkörpers (2) das Loch (6) in der Kapselkappe (1) von innen abdeckt und der Mantel der Kapselkappe (1) das Loch (7) im Kapselkörper (2) von außen abdeckt. Die Kapsel ist in dieser ersten Einsteckstellung komplett geschlossen.

In der zweiten Einsteckstellung (siehe Fig. 1c) sind Kapselkappe (1) und Kapselkörper (2) soweit ineinander geschoben, dass die jeweiligen Löcher (6) und (7) einander überdecken. Die Kapsel ist in dieser zweiten Einsteckstellung also "geöffnet" in dem Sinne, dass Pulver aus dem Inneren der Kapsel ausgebracht werden kann. Das vorgefertigte Loch (6) im hier außen liegenden Mantelbereich der Kapselkappe (1) ist größer als das Loch (7) des innen liegenden Mantelbereichs des Kapselkörpers (2). Dadurch dass eines der beiden Löcher kleiner als das andere ist, wird sichergestellt, dass auch bei Unregelmäßigkeiten beim Ineinanderstecken von Kapselkappe (1) und Kapselkörper (2) es nicht zur teilweisen Abdeckung des für die Ausbringung des Pulvers vorgesehenen Lochdurchmessers kommt. Die Größe des innenliegenden Loches (7) bestimmt in diesem bevorzugten Fall die Gesamtgröße des Lochs in der zusammengeschobenen Kapsel. Auf diese Weise kommt es auf der Innenseite der Kapsel nicht innen am Loch zu einer Stufe, an der bei Ausbringung pulverförmiges Material hängen bleiben könnte. Für die zweite Einsteckstellung sind an der Kapselkappe (1) ähnliche Rastelemente wie für die erste Einsteckstellung vorgesehen. Dementsprechend weist die Kapselkappe (1) im Darstellungsbeispiel von Figur 1 eine zweite Sicke (3) auf, in welche die Wulst (5) oder ein anderes vorspringendes Rastelement am inneren Mantelbereich des Kapselkörpers (2) einrasten kann. Dadurch werden die beiden Löcher (6) und (7) in ihrer Überdeckung gehalten und die Kapselelemente können sich auch bei Bewegung der Kapsel nicht mehr relativ zueinander verschieben. Die für die erste und zweite Einsteckstellung benötigten Rastelemente können beispielsweise durch Formgestaltung im Spritzguss von Kapselkappe (1) und Kapselkörper (2) oder durch Materialverformung an den Bauteilen realisiert werden. So kann beispielsweise eine innen im Kapselkörper umlaufende Wulst (5) mit einer außen umlaufenden Sicke einhergehen.

Zusätzlich zu der beschriebenen, als makroskopisch anzusehenden, Strukturierung der Kapselelemente weist eine weitere Ausführungsform der erfindungsgemäßen Kapsel eine Mikro- oder Nanostruktur oder Oberflächenbeschichtung innen an einem Kapselelement auf. Dies ist insbesondere jenes Kapselelement, das bei aneinanderliegenden Kapselmänteln die Außenwand der Kapsel bildet - die Kapselkappe (1) im Beispiel von Figur 1. Die Mikrostruktur befindet sich hierbei vorzugsweise innen an der dem anderen Kapselelement zugewandten Mantelfläche. Insbesondere erstreckt sich die Mikrostruktur über einen in einem ringförmigen Bereich der inneren Mantelfläche, wobei dieser ringförmige Bereich beim Vorliegen der Kapsel in der ersten Einsteckstellung (Figur 1a) eine direkte Wand des Hohlraums der Kapsel bildet und beim Vorliegen der Kapsel in der zweiten Einsteckstellung (Figur 1c) an der äußeren Mantelfläche des anderen Kapselelementes (des Kapselkörpers (2) im Beispiel von Figur 1) anliegt.
Diese Mikrostruktur bewirkt einen so genannten Lotus-Effekt, d.h. dass sie die Anhaftung von bestimmtem Material an dieser Oberfläche reduziert. Um den optimalen Effekt zu erzielen muss die Art der Mikrostruktur so gewählt sein, dass sie die geringsten Hafteigenschaften für die definierte pharmazeutische Zubereitung bietet, die in dem entsprechenden Kapseltyp bevorratet werden soll. Auf diese Weise haftet kein oder nur sehr wenig Material aus der pharmazeutischen Zubereitung, beispielsweise Pulver, an der Innenwand der Kapsel an. Dies hat insbesondere in dem beschriebenen ringförmigen Bereich den Effekt, dass es beim Zusammenschieben der Kapsel von der ersten zur zweiten Einsteckstellung nicht zu Reibung durch an der Wand anhaftendes Material kommt. Eine Ausdehnung der Mikrostruktur auf alle inneren Wandbereiche der Kapsel ist ebenfalls möglich und hat den Effekt, dass kein Material durch Wandanhaftung in der Kapsel verbleibt, wenn das Material während eines Zerstäubungsvorgangs ausgebracht wird.
Die Mikrostruktur wird durch Erhebungen und/oder Vertiefungen in der Oberfläche gebildet. Die Erhebungen und/oder Vertiefungen können die Form von Spitzen, Halbkugeln, planaren Flächen, Keilen etc. haben. Sie können eine zufällige Anordnung aufweisen oder geordnet sein, z.B. in Reihen, Kreisen, zick-zack-förmig, meanderförmig etc.
Der Abstand zwischen den Erhebungen der Oberflächenstruktur liegt im Bereich von 0,1 bis 200 Mikrometer, vorzugsweise 0,1 bis 100 Mikrometer. Bei pulverförmigen pharmazeutischen Zubereitungen sind hierbei Strukturdimensionen bevorzugt, die kleiner sind als die Korngrößen des Pulvers. Am stärksten bevorzugt sind Höhen von Erhebungen bzw. Tiefen von Vertiefungen im Bereich von 0,1 bis 50 Mikrometer und Abstände von 0,1 bis 10 Mikrometer.
Bezüglich von Verfahren zur Erzeugung einer solchen Mikrostruktur und ihrer Eigenschaften sei hiermit auf die Offenbarung der WO2004/062716 A1 auf Seite 11 Zeile 1 bis Seite 13 Zeile 13 verwiesen.
Bevorzugt werden für die Anbringung einer Mikrostruktur an Kapselinnenwänden solche Verfahren, die kein zusätzliches Material in die Kapseln hinein bringen, d.h. solche Mikrostrukturen, wie sie allein in dem das jeweilige Kapselelement bildenden Material abgeformt werden können. Im bevorzugten Fall von Kapselelementen, die in Spritzgussverfahren hergestellt werden, sind die Mikrostrukturen vorzugsweise bereits in den jeweiligen Formeinsätzen der Spritzgusswerkzeugen sozusagen spiegelbildlich abgebildet, so dass die Kapselelemente diese Mikrostrukturen wie auch die vorgefertigten Löcher bereits im ersten Fertigungsschritt erhalten. Alternativ können solche Mikrostrukturen an den Kapselinnenwänden auch durch subtraktive Oberflächenbehandlung wie Ätzen oder galvanische Abtragung oder durch nachträgliches Prägen geschaffen werden, wiebeispielsweise mittels eines aufspreizbaren Stempels, der in die Hauptöffnung des Kapselelements eingeführt wird.

Figur 2 zeigt eine zweite Ausführungsform einer aus einer Kapselkappe (1) und einem Kapselkörper (2) zusammengesetzten Kapsel. Diese Ausführungsform unterscheidet sich von der ersten nur dadurch, dass Kapselkappe (1) und Kapselkörper (2) jeweils mehrere vorgefertigte Löcher (6a) und (6b) bzw. (7a) und (7b) aufweisen. In der nicht gezeigten ersten Einsteckstellung sind alle diese Löcher durch das jeweils andere Kapselelement analog zum ersten Ausführungsbeispiel verschlossen. In der zweiten Einsteckstellung (Figur 2b) überdecken sich die jeweiligen Löcher (6a) und (6b) der Kapselkappe (1) mit den Löchern (7a) und (7b) des Kapselkörpers (2), so dass die Kapsel an mehreren Stellen geöffnet ist. Im Darstellungsbeispiel sind jeweils zwei Löcher (6a, 6b) bzw (7a, 7b) in Kapselkappe (1) und Kapselkörper (2) abgebildet, die in zwei Öffnungen an der Kapsel in der zweiten Einsteckstellung resultieren. Auf diese Weise können jedoch beliebig viele Löcher vorgesehen werden. Die Löcher können je nach Gestaltung der Mantelbereiche von Kapselkappe (1) und Kapselkörper (2) über Umfang und Länge der Kapsel verteilt werden. Besonders bevorzugt weist die resultierende geöffnete Kapsel zwei Löcher auf, die sich auf dem Mantelbereich der Kapsel jeweils nahe der entgegengesetzten Enden der Kapsel befinden, also bezogen auf die Länge der Kapsel deutlich voneinander beabstandet sind.

Figur 3 zeigt eine dritte Ausführungsform einer aus einer Kapselkappe (1) und einem Kapselkörper (2) zusammensetzbaren Kapsel. Diese Ausführungsform unterscheidet sich von der ersten nur dadurch, dass die Löcher (6) und (7) in Kapselkappe (1) und Kapselkörper (2) statt einer kreisförmigen eine elliptische Gestalt haben. Die Längsachsen dieser Ellipsen sind relativ zueinander um 90° verdreht. Auf diese Weise wird bei Unregelmäßigkeiten beim Zusammenschieben sichergestellt, dass die bei der Überdeckung zustande kommenden Öffnungen immer eine gleichbleibende Gesamtfläche einnehmen. Statt einer elliptischen Lochform können an dieser Stelle auch entsprechend ausgerichtete Langlöcher verwendet werden.

Figur 4 und 5 zeigen Kapselkörper (2) und Kapselkappe (1) schematisch im Querschnitt vor und nach dem Ineinanderstecken für verschiedene Ausführungsformen. Damit beim Ineinanderstecken von Kapselkappe (1) und Kapselkörper (2) die jeweiligen Löcher (6) und (7) sauber zur Überdeckung gebracht werden können, müssen die Kapselelemente bezogen auf ihren Umfang zueinander ausgerichtet ineinandergesteckt werden. Dies erfordert eine Struktur und Gegenstruktur ähnlich eines Schlüssel-Schloss-Prinzips an den Kanten bzw. jeweiligen Mantelflächen von Kapselkappe (1) und Kapselkörper (2). Des Weiteren ist eine Längsführung von Vorteil, die bewirkt, dass es nach gesteuertem erstem Ineinanderstecken der Kapselelemente zu keiner unerwünschten Verdrehung der Kapselelemente zueinander mehr kommen kann. In dem Ausführungsbeispiel gemäß Figur 4 sind Kapselkappe (1) und Kapselkörper (2) gleichermaßen mit leicht elliptischem Querschnitt gestaltet. Die Ellipsenform erzwingt hierbei das orientierte Ineinanderstecken der beiden Kapselelemente. Bei der Zusammenführung der elliptischen Kapselelemente ergeben sich folglich zwei mögliche, um 180° zueinander verdrehte Orientierungen. Vorzugsweise sollte daher eines der beiden Kapselelemente, besonders bevorzugt das äußere (in diesem Beispiel also die Kapselkappe (1)), nicht nur das mindestens eine vorgefertigte Loch (6) aufweisen sondern auch eine Duplizierung der Lochstruktur um 180° entlang des Umfangs des Kapselelements versetzt aufweisen. Somit wird unabhängig von der Orientierung beim Zusammenführen zweier elliptischer Kapselelemente immer ein Loch (6) beim Ineinanderschieben in die zweite Einsteckstellung freigegeben.
In dem Ausführungsbeispiel gemäß Figur 5 weist der Außenbereich des Kapselkörpers (2) zusätzlich zu den nicht gezeigten Rastelementen einen parallel zur Längsachse verlaufenden Steg (8) auf, dessen Kontur zu einer ebenfalls längs, innen an der Kapselkappe (1) angebrachten Nut (9) passt. Die Kapselelemente lassen sich nur in der azimutalen Ausrichtung, in welcher der Steg (8) und die Nut (9) ineinander greifen, zusammen schieben. Steg (8) und Nut (9) bilden eine Führung über die gesamte Einschublänge. Wahlweise können auch mehrere unterschiedlich zueinander beabstandete und/oder unterschiedliche breite Steg-und-Nut-Paare an den Mantelflächen der Kapselelemente angeordnet sein.
In einer nicht dargestellten Ausführungsform kann das gezeigte Strukturpaar aus Steg (8) und Nut (9) auch eine Kurvenbahn aufweisen, durch welche ein Zusammenschieben der Kapselelemente eine Drehung derselben relativ zueinander erzwingt. Dies kann insbesondere bei dem gezielten Verdecken und Freilegen von mehreren Löchern in der Kapsel von Vorteil sein.

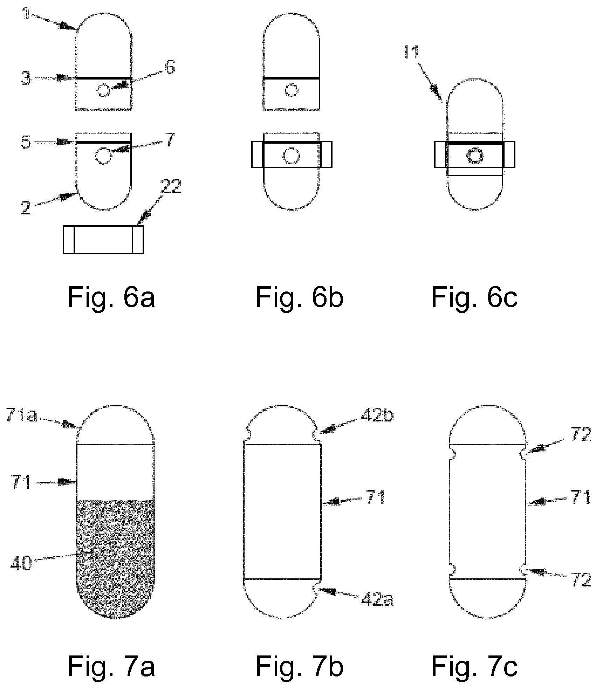

Fig. 6 zeigt in schematischer Darstellung eine weitere Ausführungsform einer erfindungsgemäßen Kapsel bestehend aus Kapselkappe (1), Kapselkörper (2) und einem Ring (22). Kapselkappe (1) und Kapselkörper (2) haben analog zu dem Ausführungsbeispiel von Figur 1 beide ebenfalls eine becherartige Form und können teleskopartig über ihre Öffnungen ineinander gesteckt werden, wobei diesbezüglich anhand von Figur 1 beschriebene Aspekte hier ebenfalls ihre Gültigkeit haben. Aus Gründen der einfacheren Befüllung ist im Darstellungsbeispiel von Fig. 6 abweichend zu den anderen Darstellungsbeispielen eine Ausführungsform gewählt worden, bei der die Kapselkappe (1) in den Kapselkörper (2) eingeschoben wird (Ausführungsformen mit außen aufgeschobener Kappe sind aber ebenfalls möglich). Der Kapselkörper (2) mit gegenüber der Kapselkappe (1) vergrößertem Loch (7) bildet hier also die äußere Wand der Kapsel im Bereich der zwei aufeinander liegenden Mantelbereiche der Kapselelemente, die Kapselmantelbereiche der beiden Kapselelemente weisen analog zu den vorherigen Beispielen miteinander wechselwirkenden Strukturierungen auf.
Fig. 6a zeigt die beiden getrennten Kapselelemente (1) und (2) mit den vorgefertigten Löchern (6) und (7) vor dem Ineinanderstecken und vor Zusammenfügen mit dem Ring (22). Fig. 6b zeigt den Kapselkörper (2) nach Zusammenfügen mit dem Ring (22). Der Ring (22) verdeckt hierbei das Loch (7) des Kapselkörpers (2). Dies ist vorzugsweise die Situation, in der die pharmazeutische vorzugsweise pulverförmige Zubereitung in den Kapselkörper (2) eingefüllt wird, der dann wie in Fig. 6c gezeigt mit der Kapselkappe (1) verschlossen wird. Die Kapselkappe (1) kann bei diesem Konzept direkt bis zum Erreichen der finalen Einsteckstellung in den Kapselkörper (2) geschoben werden.
Fig. 6c zeigt die zusammengesetzte Kapsel (11), in der analog zum Ausführungsbeispiel von Figur 1 Kapselkappe (1) und Kapselkörper (2) mittels Rastelemente (3) und (5) miteinander verrastet sind. Die jeweiligen vorgefertigten Löcher (6) und (7) überdecken sich bei dieser zusammengesetzten Kapsel, das resultierende Loch wird in diesem Zustand jedoch vom Ring (22) verdeckt. Die Kapsel (11) kann so gelagert oder in einen geeigneten Inhalator ein gesetzt werden. Alternativ zu der hier gezeigten Kapsel (11) können bei diesem Konzept mit Abdichten der vorgefertigten Löcher (7) über ein weiteres z.B. ringförmiges Bauteil die Kapselelemente auch so gestaltet werden, dass sich nach Zusammensetzen nicht zwangsläufig jeweils ein Loch (7) in Kapselkörper (2) mit einem Loch (6) in Kapselkappe überdecken muss, sondern dass jeweils der Kapselmantel des anderen Kapselelements im zusammengeschobenen Zustand der Kapsel (11) so kurz ist, dass er das jeweilige Loch frei lässt. Bei diesem alternativen (nicht gezeigten Ausführungsbeispiel) weist der Kapselkörper (2) ein vorgefertigtes Loch (7), die Kapselkappe (1) hat zu diesem Loch (7) passendes Loch (6) und der Mantel der Kapselkappe (1) endet im zusammengeschobenen Zustand der Kapsel oberhalb des Loches (7) und lässt dieses frei.

Fig. 7 zeigt in schematischer Darstellung weitere Ausführungsform einer Kapsel. In Fig. 7 a wird die Kapsel als Ganzes mit Befüllung gezeigt. Die Kapsel ist im Wesentlichen zylindrisch mit halbkugelförmigen oberen und unteren Enden. Die geschlossene Kapsel (71) weist in ihrem Inneren eine abgemessene Dosis des Pulvers (40) auf.
In Fig. 7b sind Löcher 42a und 42b im halbkugelförmigen oberen Ende bzw. Deckel und im unteren Ende bzw. Boden der Kapsel platziert worden. Für die Ausbringung des Pulvers insbesondere unter Ausnutzung des Bernoulli-Effekts in einem Inhalator wird zumindest ein Loch wird im Boden und zumindest eines im Deckel der Kapsel benötigt. Es können auch jeweils mehr als ein Loch in Boden und Deckel verwendet werden. Die Lochgrößen haben vorzugsweise Durchmesser zwischen 0,01 und 5 Millimetern, bevorzugt zwischen 0,5 und 1,5 Millimeter. Die Löcher sind vorzugsweise kreisförmig, können aber auch oval, quadratisch oder von sonstiger Gestalt sein. Die Löcher können im Spritzgießprozess abgeformt, mit konventionellen Bohrern oder mit Lasern gebohrt, gestempelt oder auf sonstige Weise gebildet werden, bevor die Kapsel mit Pulver befüllt wird.
In Fig. 7c sind die Löcher (72) in den parallelen Wänden bzw. im zylindrischen Mantelbereich der geschlossenen Kapsel (71) platziert worden. Auf diese Weise kann das Pulver einfacher in der Kapsel mit vorgefertigten Löchern gelagert werden, da die Kapsel passgenau in einer zylindrischen Röhre gelagert werden kann, welche die Kapsel dann im Mantelbereich dicht umschließt.
Fig. 7b und Fig. 7c zeigen lediglich Varianten, in denen die Löcher (42a, 42b bzw. 72) entweder in den halbkugelförmigen oder in den zylindrischen Bereichen der Kapsel (71) ausgebildet sind. Darüber hinaus sind jedoch auch Varianten einer Kapsel (71) mit insgesamt mindestens zwei Löchern (42a, 42b bzw. 72) möglich, bei denen sich das Loch oder die Löcher (42a, 42b bzw. 72) auf einer Seite der Kapsel (71) im halbkugelförmigen Bereich und das Loch oder die Löcher (42a, 42b bzw. 72) auf der anderen Seite der Kapsel (71) im zylindrischen Mantelbereich befindet oder befinden. Die mindestens zwei Löcher (42a, 42b bzw. 72) können des Weiteren bezogen auf den kreisförmigen Umfang der Kapsel (71) auch um 180° oder andere Winkeleinheiten versetzt angeordnet sein. Ebenfalls sind zusätzlich viele Variationen hinsichtlich der Verteilung von unterschiedlich vielen Löchern (42a, 42b bzw. 72) an der Kapsel (71) möglich, beispielsweise kann sich auf einer Seite (unten bezogen auf Fig. 7b) nur ein Loch (42a, 42b bzw. 72) und auf der anderen Seite (oben) mindestens zwei Löcher (42a, 42b bzw. 72) befinden.

Fig. 8 zeigt schematisch die Funktionsweise eines Geräts (70) bzw. Inhalators, in dem eine Kapsel (71) (Fig. 8a) mit zwei vorgefertigten Löchern (72a, 72b) in dem Gerät so bevorratet ist, dass die Löcher (72, 72b) im Lagerzustand (Fig. 8b) verschlossen sind, und die Kapsel (71) für die Anwendung des Geräts aus dem Lagerzustand in eine Kapselkammer (74) verschoben wird.
Fig. 8a zeigt die ansonsten geschlossene Kapsel (71) mit vorgefertigten Löchern (72a, 72b). Die Kapsel (71) ist im Wesentlichen zylindrisch mit halbkugelförmigen Enden. (71a, 71b). Die Löcher (72a, 72b) befinden sich in den parallelen Wänden der Kapsel bzw. im Mantelbereich der Kapsel jeweils nahe der halbkugelförmigen Enden. Analog können in diesem Zusammenhang auch Kapseln gemäß anderer Ausführungsformen eingesetzt werden, insbesondere entsprechend der in Fig. 7c gezeigte Kapsel.
Fig. 8b zeigt die Kapsel in ihrem Lagerzustand in einem Gerät (70). In diesem Lagerzustand wird die Kapsel (71) fest in einer Röhre (73) gehalten. Dies verhindert den Austritt von Pulver aus der Kapsel (71), da die innere Wand der Röhre die Löcher (72a, 72b) abdeckt. Innerhalb des Geräts (70) ist eine Kapselkammer (74) ausgebildet. Die Kapselkammer (74) schließt direkt an die Röhre (73) an und hat einen etwas größeren vorzugsweise kreisförmigen Durchmesser als die Röhre (73). Die Kapselkammer (74) wird durch einen Stab (75) oder durch ein anderes vorzugsweise aerodynamisch geformtes Bauteil im Luftausgangsbereich begrenzt.
In Fig. 8c ist das Gerät (70) anwendungsbereit, d.h. es befindet sich im Anwendungszustand. Die Kapsel (71) ist durch den Einlass (76) in die Kapselkammer (74) geschoben worden. Hierzu ist auf der der Kapselkammer (74) entgegengesetzten Seite der Kapsel (71) ein kolbenförmiger Schieber in die Röhre (73) eingeführt. Die Schiebefläche des Schiebers begrenzt im Anwendungszustand die Kapselkammer (74) auf der dem Luftausgang entgegengesetzten Seite. Zur Anwendung atmet der Anwender durch ein Mundstück (78) in Richtung des Pfeils (92) in der Abbildung ein. Luft gelang in das Gerät (70) entlang der Richtung des Pfeils (91) in der Abbildung durch den Einlass (76), bzw. durch eine Luftführung im Schieber, der vorzugsweise die Gestalt eines Hohlkolbens hat. Im Luftstrom vibriert die Kapsel (71) in der Kapselkammer (74), wobei das Pulver über die Löcher (72a, 72b) aus der Kapsel (71) ausgetrieben wird.

In einer anderen nicht dargestellten erfindungsgemäßen Ausführungsform lagert die Kapsel schon in der Vibrationskammer bzw. Kapselkammer (74) und die Kapsel (71) ist mit einer vorzugsweise schlauchförmig ausgebildeten Folie umzogen. Die Folie liegt dicht am zylindrischen Mantelbereich der Kapsel (71) an und verschließt die Löcher (72). Vorzugsweise können dabei die Materialien von Folie und Kapselwand so gewählt sein, dass sich die vorzugsweise elastische und/oder leicht biegbare Folie durch elektrostatische Anziehung an die Kapselwand anschmiegt. An einem Endpunkt ist die Kapsel (71) vorzugsweise nicht fest von der Folie umschlossen und an der anderen Seite ragt sie deutlich über die Kapsel (71) hinaus und/oder ist mit einem Zugband verbunden. Dieser am Kapselende hinaus ragende Folienteil und/oder das Zugband liegt im Transportzustand des Inhalators derart im Lufteinlass, dass an dieser Stelle ein Teil der Folie und/oder eines Zugbandes aus dem Inhalator herausragt. Vor der Anwendung des Inhalators wird die Folie an diesem herausragenden Teil und/oder an diesem Zugband durch den Lufteinlass von der Kapsel, die nicht durch den Lufteinlass passt, abgezogen. Beispielsweise befindet sich im Lufteinlass ein Stab (75) oder ein anderes Hindernis, das die Kapsel (71) aufhält (in diesem Beispiel liegt die Folie bzw. der Folienschlauch nur auf einer Seite des Stegs im Luftkanal). Wenn die Folie bzw. der Folienschlauch weggezogen, aber die Kapsel aufgehalten wird, gleitet die Kapsel aus der vorzugsweise flexiblen Folie bzw. dem Folienschlauch. Dadurch werden die Löcher (72) freigegeben, die Kapsel (71) erhält ihren vollen Bewegungsspielraum in der Kapselkammer (74), und der Inhalator ist anwendungsbereit. Alternativ kann der überstehende Teil der Folie bzw. das Zugband auch im Mundrohr des Inhalators liegen und durch das Mundrohr aus dem System herausgezogen werden. Generell kann die Öffnung, durch welche die Folie aus dem System gezogen wird im Transportzustand auch verschlossen sein. In der Ausführungsform, in der der überstehende Folienteil bzw. das Zugband im Mundrohr liegt, kann beispielsweise das Mundrohr durch eine am Mundstück (78) angebrachte Kappe verschlossen sein, die erst abgenommen werden muss, bevor man die Folie heraus ziehen kann. Eine solche Kappe kann auch direkt mit dem Zugband oder der Folie verbunden sein.

Fig. 9 zeigt schematisch den Aufbau einer weiteren Ausführungsform eines Systems aus Inhalator und Kapsel, wobei die Funktionsweise des Systems ähnlich ist wie bei dem in Fig. 8 gezeigten System. Die Teilbilder 9a bis 9f stellen dabei gleichzeitig auch schematisch den Ablauf einer Montage des Systems dar. Zunächst (Fig. 9a) wird ein Kapselkörper (2) mit mindestens einem vorgefertigten Loch in einen Ring (22) eingesetzt, der den Kapselkörper (2) im Bereich seines mindestens einen vorgefertigten Loches passgenau umschließt und somit das Loch abdichtet. Optional weist der Ring (22) innen einen oder vorzugsweise mehrere kleine Vorsprünge auf, die beim Zusammenfügen von Kapselkörper (2) und Ring (22) eine untere Position des Kapselkörpers (2) im Ring (22) vorgegeben, so dass der Kapselkörper (2) sich innerhalb des Rings nicht nach unten verschieben kann und/oder zwischen Kapselkörper (2) und Ring (22) eine Passung ausgebildet wird, durch die der Kapselkörper (2) im Ring (22) zurückgehalten wird. Den Ring (22) betreffende Merkmale aus diesem Ausführungsbeispiel sind analog auch auf den Ring (22) aus dem Ausführungsbeispiel gemäß Fig. 6 übertragbar.
Fig. 9b zeigt wie der Kapselkörper (2) im Ring (22) von unten in eine Röhre (73) eingesetzt wird. Die Röhre (73) bildet in diesem Ausführungsbeispiel die Kapselaufnahme. Bevorzugt verrastet der Ring (22) dabei von innen mit der Röhre (73) in einer ersten Raststellung, z.B. indem eine umlaufenden Wulst (22a) oder ein anderweitig geformtes Rastelement von innen in der Röhre (73) in eine entsprechende Aussparung eingreift. Die Röhre (73) ist an ihrem oberen Ende geöffnet, so dass der Kapselkörper (2), dessen Öffnung bei Einsetzen in die Röhre (73) nach oben zeigt, von oben durch die obere Öffnung der Röhre (73) mit Pulver (40) befüllt werden kann. Fig. 9c zeigt dann, wie - nach der nicht dargestellten Befüllung mit Pulver (40) - die Kapselkappe (1), die vorzugsweise ebenfalls mindestens ein vorgefertigtes Loch aufweist, von oben in die Röhre (73) eingesetzt wird und so im Inneren der Röhre (73) den Kapselkörper (2) verschließt, bzw. die Kapsel (71) in der Röhre (73) zusammengesetzt wird. Die Röhre (73) umschließt dabei die Kapselkappe (1) vorzugsweise mit einem oberen Kragen (73a) passgenau, so dass das mindestens eine Loch (72b) in der Kapselkappe (1) durch die Röhre (73) verdeckt bzw. abgedichtet ist.
Fig. 9d und Fig. 9e zeigen, wie die weiteren Bauteile des Inhalators - Mundstück (78) und Schieber (77) - mit der Baugruppe bestehend aus Röhre (73), pulvergefüllter Kapsel (71) und Ring (22) zusammengesetzt werden: Das Mundstück (78), das vorzugsweise einstückig ausgebildet ist, enthält die Kapselkammer (74) und einen Stab (75) als Begrenzung oben im Innern der Kapselkammer (74). Das Mundstück wird von oben auf die Röhre (73) aufgesetzt, wobei es mit der Röhre (73) verrastet. Hierzu sind miteinander wechselwirkende Rastelemente an Mundstück (78) und Röhre (73) ausgebildet, z.B. in Form einer unten im Inneren des Mundstücks angebrachten, ringförmig umlaufenden Wulst (78b), die in eine ebenfalls ringförmig umlaufende Sicke (73b) außen, oben an der Röhre (73) einrastet. Auf der dem Mundstück entgegengesetzten Seite, d.h. in der Darstellung von unten, wird ein Schieber (77) in die Röhre (73) eingesetzt. Der Schieber (77) beinhaltet einen Einlass (76) durch den später bei Anwendung des Inhalators Luft in die Kapselkammer (74) einströmen kann. Vorzugsweise ist der Schieber (77) dabei als Hohlkolben ausgebildet und/oder der Einlass (76) wird durch eine radialsymmetrische Durchführung entlang der Hauptachse des Schiebers (77) gebildet. Der Schieber (77) weist an seinem oberen Ende eine Verjüngung (77a) auf, die derart bemessen ist, dass sie in den Ring (22) eintauchen kann.
Fig. 9f zeigt im schematischem Längsschnitt das fertig zusammen gesetzte System aus Kapsel (71) und Inhalator im Transportzustand: Die Kapsel (71) ist derart im Inhalator vorinstalliert, dass ihre vorgefertigten Löcher (72a, 72b) vom Ring (22) und/oder vom Inneren der Röhre (73) verdeckt bzw. abgedichtet werden (im dargestellten Ausführungsbeispiel mit insgesamt zwei vorgefertigten Löchern (72a, 72b) wird ein Loch (72a) vom Ring (22) und ein Loch (72b) vom Inneren der Röhre abgedichtet). Der Schieber (77) ragt mit seinem unteren Ende aus dem Inhalator heraus. Zur Aktivierung des Inhalators, d.h. zur Überführung des Geräts aus dem Transportzustand in den Anwendungszustand wird der Schieber an seinem unten am Gerät vorstehenden Ende in das Gerät hinein gedrückt. Vorzugsweise ist hierzu das untere Ende des Schiebers (77) derart verbreitert, dass es beim Drücken mit der Hand angenehm am Handballen des Anwenders bzw. Patienten anliegt. Beim Hineinschieben stößt der Schieber (77) vorzugsweise mit einer ringförmig gestalteten Kontaktfläche von unten gegen die Kapsel (71) und schiebt diese - bedingt durch ihre Rückhaltung im Ring (22) - zunächst zusammen mit dem Ring (22) weiter in Richtung Kapselkammer (74), bis der Ring (22) in einer zweiten Raststellung verrastet. Beim weiteren Hineinschieben des Schiebers (77) in die Röhre (73) taucht der Schieber (77) in den Ring (22) ein und drückt mit der Verjüngung (77a) die Kapsel (71) aus ihrer passgenauen Halterung im Ring (22) in die Kapselkammer (74). Die Verjüngung (77a) ist dabei vorzugsweise so lang ausgelegt, dass der verjüngte Bereich des Schiebers (77) durch den Ring (22) und durch den Kragen (73a) der Röhre (73) hindurch geschoben werden kann, bis die Oberkante des Schiebers (77) die untere Begrenzung der Kapselkammer (74) bildet. Vorzugsweise schließt die Oberkante des Schiebers (77) im eingeschobenen Zustand (Anwendungszustand des Inhalators, siehe Fig. 9g) bündig mit der Oberkante der Röhre (73) ab. Der Spalt, der sich je nach Auslegung des Systems - z.B. bedingt durch einen im Vergleich zu Kapselkörper (2) und Verjüngung (77a) größeren Durchmesser der Kapselkappe (1) - im Boden der Kapselkammer (74) zwischen dem oberen Rand des Schiebers (77) und dem Kragen (73a) oben an der Röhre (73) bildet, wird hierbei vorzugsweise durch den Ring (22) gegen das Eindringen von Nebenluft abgedichtet. Der Ring (22) besteht hierfür aus einem zumindest teilweise elastischen Material und dichtet in diesem eingeschobenen Zustand (siehe Fig. 9g) des Schiebers (77) den Schieber (77) gegen die Röhre (73) unterhalb des Kragens (73a) ab.
Der Inhalator ist dann, wie in Fig. 9g gezeigt, funktionsbereit: die Löcher (72a, 72b) sind freigelegt und die Kapsel (71) hat in der Kapselkammer (74) den Bewegungsspielraum, der für die Vibrationsbewegung gemäß dem Bernoulli-Effekt benötigt wird. Vorzugsweise sind seitlich am Schieber (77) mindestens ein, vorzugsweise zwei Federarme (77b) ausgebildet, die im eingeschobenen Zustand in entsprechende Ausnehmungen (73s) innen an der Röhre (73) eingreifen. Diese Federarme (77b) verhindern durch ihren Eingriff innen in der Röhre ein Herausziehen des Schiebers (77) aus dem Gerät heraus. Dem Anwender wird somit verdeutlicht, dass es sich hierbei um ein Gerät für eine einmalige Anwendung handelt.

Fig. 10 zeigt schematisch den Aufbau einer erfindungsgemäßen Ausführungsform eines Systems bestehend aus Inhalator und einer pulvergefüllten Kapsel (71), wobei insbesondere eine Kapsel gemäß der in Fig. 7c oder Fig. 8 gezeigten Ausführungsformen eingesetzt werden kann. Die Teilbilder 10a bis 10e stellen dabei gleichzeitig schematisch den Ablauf einer Montage des Systems dar.
Zunächst (Fig. 10a) wird ein Kapselkörper (2) mit mindestens einem vorgefertigten Loch von oben in eine in diesem Ausführungsbeispiel im Wesentlichen nur einseitig offene Röhre (73) eingesetzt, welche die Kapselaufnahme bildet. Die Röhre (73) umschließt den Kapselkörper (2) im Bereich seines mindestens einen vorgefertigten Loches passgenau und dichtet somit das Loch im Kapselkörper (2) ab. Die einseitige Öffnung des Kapselkörpers (2) zeigt dabei nach oben, d.h. in jene Richtung, aus der der Kapselkörper (2) in die Röhre (73) eingesetzt wurde.
Je nach Gestaltung der Kapselaufnahme ist es vorteilhaft, die Kapsel (71) orientiert in diese einzusetzen. In der hier dargestellten Ausführungsform weist die Kapselaufnahme beispielsweise zwei längs verlaufende Schlitze (73d) auf, deren Funktion anhand der Beschreibung von Fig. 10f näher erläutert wird. Um eine Abdichtung der Löcher (72a, 72b) durch das Einsetzen der Kapsel (71) in die Kapselaufnahme zu gewährleisten, wird die Kapsel (71) bevorzugt derart orientiert in die Röhre (73) eingesetzt, dass sich die Löcher (72a, 72b) nicht im Bereich der Schlitze (73d) befinden. Um eine Orientierung der Kapsel (71) beim Einsetzen in die Kapselaufnahme vorzugeben, können die anhand von Fig. 4 und Fig. 5 beschriebenen Techniken angewandt werden. So können Kapsel (71) und das Innere der Kapselaufnahme beispielsweise leicht elliptisch hinsichtlich ihres kurzen Durchmessers ausgebildet sein. Alternativ können Kapsel (71) oder das Innere der Kapselaufnahme entsprechende Paarungen von längsverlaufenden Stege und Rillen aufweisen: beispielsweise eine Längsnut oder längsverlaufende Rille an der äußeren Wand der Kapsel (71) in Kombination mit einem längs ausgebildeten Steg im Inneren der Kapselaufnahme.
Fig. 10b zeigt die Befüllung des Kapselkörpers (2) innerhalb der Röhre (73) mit Pulver (40), das von oben in die einseitige Öffnung des Kapselkörpers (2) eingefüllt wird. Anschließend (Fig. 10c) wird aus gleicher Richtung, d.h. in der Darstellung von oben, die Kapselkappe (1) in die Röhre (73) eingeführt, so dass die Kapsel (71) innerhalb der Röhre (73) verschlossen wird. Fig. 10d zeigt, wie die weiteren Bauteile des Inhalators - Mundstück (78) und Stab (75) - mit der Baugruppe bestehend aus Röhre (73) und pulvergefüllter Kapsel (71) zusammengesetzt werden: Die Röhre (73) wird (nun in Fig. 10d im Vergleich zu Fig. 10c kopfüber dargestellt) von oben durch eine Öffnung am Mundstück (78) vorzugsweise bis zu einem von der Kapselkammer (74) gebildeten unteren Anschlag eingeschoben. Die Seite, von der die Röhre (73) eingeführt wird, ist hierbei auch die Mundseite des Mundstücks, d.h. die Seite an der der Patient zum Gebrauch des Inhalators die Lippen ansetzt. Nach Einsetzen der Röhre (73) in das Mundstück (78) wird ein Stab (75) durch eine seitlich am Mundstück befindliche Durchführung eingeschoben. Der Stab (75) bildet später die obere Begrenzung der im Mundstück (78) befindlichen Kapselkammer (74). Die Röhre (73) ist vor dem Einsetzen des Stabs (75) derart orientiert in das Mundstück eingesetzt worden, dass zwei an der Röhre vorgesehene Schlitze (73d) an der Durchführung im Inneren am Mundstück (78) anliegen und somit Platz für den Einschub des Stabs (73) lassen. Auf diese Weise kann der Stab (75) so in das Mundstück (78) eingeführt werden, dass er ohne Behinderung durch die Röhre (73) das Mundstück (78) vorzugsweise quer durch die Hauptachse des Geräts hindurch das Mundstück (78) von einer Außenwand zur anderen durchdringt. Das orientierte Einsetzen der Röhre (73) in das Mundstück (78) wird vorzugsweise durch die äußere Form der beiden Bauteile vorgegeben. So zeigt das Darstellungsbeispiel beispielsweise ein Mundstück (78) mit einem im Wesentlichen ovalen oder trapezförmigen Querschnitt aus der Mundseite des Mundstücks (78). Die Röhre (73) ist in ihrem oberen - von der Kapselaufnahme entfernten - Bereich vorzugsweise in Form einer Kappe ausgebildet, die das Mundstück an seinem mundseitigen Ende komplett abdeckt. Diese Kappenstruktur der Röhre (73) bildet das Gegenstück zu der im Wesentlichen ovalen oder trapezförmigen Gestalt der Mundseite des Mundstücks (78), so dass eine Orientierung beim Zusammenführen durch die Ovalität bzw. die Vorzugsachse einer nicht kreisförmigen Symmetrie vorgegeben ist. Die in Fig. 10 gezeigte Kappenstruktur der Röhre (73) bietet auch zusätzlich den Vorteil, dass an der Mundseite die Außenflächen des Mundstücks (78) in den Bereichen, in denen der Patient seine Lippen ansetzt, im Transportzustand von der Röhre (73) verdeckt werden. Somit wird selbst bei sehr frühzeitiger Entnahme des Geräts aus einer Umverpackung gewährleistet, dass die Bereiche für den Lippenkontakt bis zur Anwendung des Geräts frei von Verschmutzungen bleiben.
Der Stab (75) bildet die obere Begrenzung der Kapselkammer (74) im Innern des Mundstücks (78). Außer dem Stab (75) werden alle anderen Bestandteile der Kapselkammer (74) einstückig vom Mundstück (78) gebildet. Am unteren Ende der Kapselkammer (74), d.h. an ihrem dem Stab (75) und der Mundstücköffnung gegenüber liegenden Ende, weist das Mundstück einen Einlass (76) auf, der im Darstellungsbeispiel als mittige Durchführung entlang der Hauptachse des Systems ausgebildet ist.
Der hier dargestellte Inhalator besteht vorzugsweise nur aus drei Teilen - Mundstück (78), Röhre (73) und Stab (75), die alle kostengünstig in Kunststoff-Spritzguss-Verfahren hergestellt werden können, so dass ein derart aufgebauter Inhalator sich sehr gut zur Einmal-Anwendung eignet, d.h. als Wegwerf-Artikel nach einmaliger Anwendung vorgesehen werden kann.
Fig. 10e zeigt das System aus Inhalator und Kapsel (71) im zusammengesetzten Zustand, der hier auch dem Transportzustand entspricht. Die (nicht gezeigten) vorgefertigten Löcher (72a, 72b) der Kapsel (71) werden durch die Innenwand der Röhre (73) verschlossen. Für Kapseln (71), von denen ein Kapselelement z.B. die Kapselkappe (1) einen größeren Außendurchmesser als der zugängliche Teil des anderen Kapselelementes z.B. des Kapselkörpers (2) hat, weist die Röhre (73) eine entsprechende Veränderung des Innendurchmessers auf: das Kapselelement mit geringerem Durchmesser befindet sich weiter im Innern der Röhre (73) als das Kapselelement mit größerem Durchmesser, und der innere Durchmesser der Röhre (73) ist entsprechend an die äußere Gestalt der Kapsel (71) angepasst, so dass auch er von innen nach außen schrittweise breiter wird. Im Transportzustand ist die Kapsel (71) somit ohne nennenswerten Bewegungsspielraum im System eingeschlossen. Für einen festen Sitz der als Kappe gestalteten Röhre (73) auf dem Mundstück (78) weist die Röhre in ihrem oberen Bereich vorzugsweise Federarme (73f) auf, die sich im Transportzustand von innen gegen die Innenwand des Mundrohrs (78d) drücken, das auf der Mundseite des Mundstücks (78) den Luftauslass aus der Kapselkammer (74) bildet. Vorzugsweise haben Mundrohr (78d) und Kapselkammer (74) den gleichen Durchmesser.
Zur Anwendung des Inhalators wird dann (wie in Fig. 10f) gezeigt die Röhre (73) aus dem Mundstück (78) herausgezogen. Die Schlitze (73d) in der Röhre (73) ermöglichen hierbei das Herausziehen der Röhre (73) am Stab (75) vorbei. Der in das Mundstück (78) eingesetzte und sich quer durch die Mundstücköffnung erstreckende Stab (75) bewirkt, dass die Kapsel (71) in der Kapselkammer (74) im Mundstück (78) verbleibt und nicht mit der Röhre (73) wieder herausgezogen werden kann. Wird die Röhre (73) aus dem Mundstück (78) herausgezogen, so spreitzen sich die zuvor im Mundrohr (78d) zusammengedrückten Federarme (73f) vorzugsweise so nach außen auf, dass die Röhre (73) nicht wieder (ohne Hilfsmittel) zerstörungsfrei in das Mundstück (78) eingesetzt werden kann. Auf diese Weise wird dem Anwender verdeutlicht, dass es sich bei diesem Gerät um einen Wegwerf-Artikel bzw. ein Ein-Weg-Produkt handelt. Um dem Anwender das Herausziehen der Röhre (73) aus dem Mundstück (78) zu erleichtern, weist die Röhre (73) vorzugsweise eine Griffhilfe (73e) auf. Diese Griffhilfe kann beispielsweise wie in Fig. 10d gezeigt durch eine Riffelung der äußeren seitlichen Oberfläche im Kappenbereich der Röhre (73) oder (nicht gezeigt) durch eine Lasche gebildet werden. Diese Lasche befindet sich vorzugsweise mittig oben im Kappenbereich der Röhre (73), also an der Seite, die der Einführöffnung für die Kapsel (71) entgegengesetzt ist, und beinhaltet eine Öffnung, die derart bemessen ist, dass der Anwender einen Finger (vorzugsweise seinen Zeigefinger) in die Öffnung einhaken kann, um anschließend die Röhre (73) aus dem Mundstück (78) zu ziehen. Damit gleichzeitig das Mundstück bequem mit Fingern der anderen Hand gehalten werden kann, ist es vorzugsweise mit einer Grifffläche (78c) ausgestattet, die in dem Moment des Rausziehens der Röhre (73) ein Wegrutschen des Mundstücks (78) in der Hand bzw. zwischen den Fingern verhindert. Diese Grifffläche (78c) wird vorzugsweise durch eine Mehrzahl von abgerundeten Erhebungen oder Noppen gebildet, die sich insbesondere mittig außen auf zwei Seiten am Mundstück (78) unterhalb des vorzugsweise verbreiterten Bereichs zum Ansetzen der Lippen befindet.

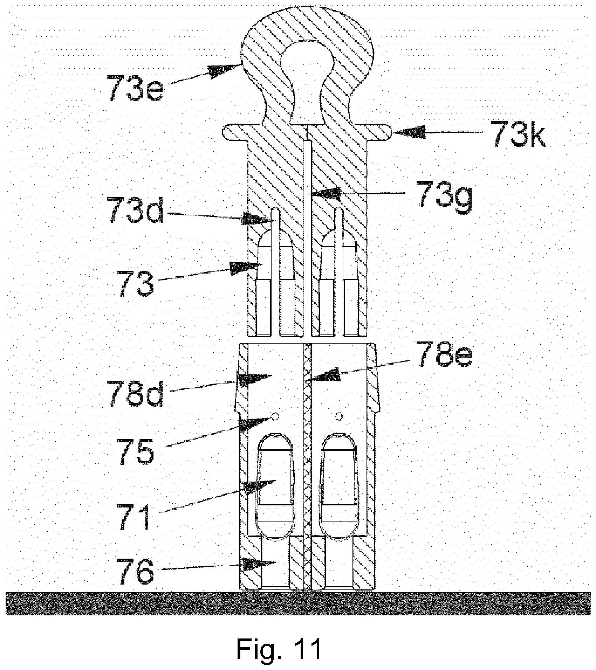

Fig. 11 zeigt ein System aus einem Inhalator und zwei Kapsel (71). Die Funktionen in diesem Ausführungsbeispiel entsprechen denen des Ausführungsbeispiels aus Fig. 10 mit dem Unterschied, dass zwei Kapseln (71) in individuellen Kapselkammern (74) vorgesehen sind. Alle mit den Kapseln (71) wechselwirkende Merkmale sind somit doppelt ausgelegt: Der Inhalator weist zwei Kapselkammer (74) mit zwei Einlässen (76), zwei Stäben (75) und zwei Röhren (73) mit Schlitzen (73d) auf. Im Transportzustand umschließen die Wände der Röhren (73) die Kapseln (71) derart, dass die vorgefertigten Löcher (72) in den Kapseln verschlossen sind. Die beiden Röhren (73) sind in der abgebildeten Ausführungsform vorzugsweise in einem Abschlussbereich (73k) so verbunden, dass sie Teil einer Kappe sind, die analog zum Ausführungsbeispiel aus Fig. 10 die Öffnung des Mundstücks (78) im Transportzustand verschließt. An dieser Kappe ist eine Griffhilfe (73e) ausgebildet, die in Fig. 11 in der Ausführungsform als Lasche mit Öffnung dargestellt ist. Vor der Inhalation zieht der Anwender die Kappe an der Lasche vom Mundstück (78) weg, erhält so den in Fig. 11 dargestellten Zustand. Die Löcher (72) an den Kapseln (71) sind dann nach der Entnahme der Röhren (73) aus den Kapselkammern (74) geöffnet und die Kapseln (71) haben den für die gewünschte Vibration benötigten Bewegungsspielraum. Die Strömungswege durch die beiden Kapselkammern sind vorzugsweise nicht miteinander verbunden. Der Inhalator weist also zwei Mundrohre (78d) auf, die durch eine Mittelwand (78e) von einander getrennt sind. Beide Mundrohre (78d) verlaufen parallel und öffnen sich nebeneinander in einem vorzugsweise außen oval gestalteten Mundstück (78). Die beiden Röhren (73) sind im Transportzustand des Geräts passgenau in die beiden Mundrohre (78d) eingesteckt, wobei ein Abstand (73g) zwischen den Röhren vorgesehen ist, um Raum für die Mittelwand (78e) zu geben.
Die beiden Kapseln (71) können je nach Anwendungszweck des Inhalators identisch oder hinsichtlich ihrer Befüllung und/oder äußeren Gestalt verschieden sein. Die Verwendung eines Geräts mit zwei identischen Kapseln (71) hat den Vorteil, dass man mit einem einzigen Wegwerf-Gerät die doppelte Formulierungsdosis ausgeben kann und somit Wegwerfmaterialkosten spart. Die Verwendung eines Geräts, in dem zwei verschieden befüllte Kapseln (71) bevorratet sind, eignet sich vor allem für den Einsatz in Therapien, bei denen zwei Wirkstoffe gleichzeitig verabreicht werden, die unter Umständen nicht stabil in einer gemeinsamen Formulierung gelagert werden können. Mit einem solchen Gerät wird sichergestellt, dass beide Wirkstoffe in richtigem Mengenverhältnis zueinander eingenommen werden. Es wird somit beispielsweise ausgeschlossen, dass ein Anwender zweimal zum gleichen Präparat statt zu zwei verschiedenen Präparaten bei einem Einnahmezyklus greift.
Die Kapseln (71) und ihre zugehörigen Kapselkammern (74), Mundrohre (78d) und Röhren (73) können für diesen Zweck an Wirkstoff bzw. Formulierungsdosis angepasst sein, beispielsweise unterschiedlich groß sein bzw. unterschiedliche Durchmesser und/oder Längen haben.

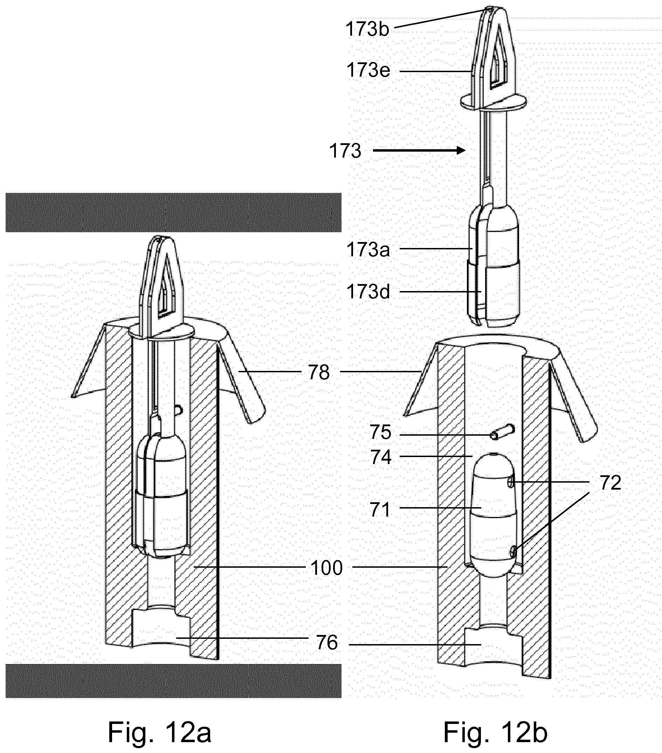

Fig. 12 zeigt schematisch in angeschnittener Darstellung den Aufbau einer weiteren erfindungsgemäßen Ausführungsform eines Systems bestehend aus Inhalator und einer pulvergefüllten Kapsel (71), wobei insbesondere eine Kapsel gemäß der in Fig. 1-5, 7c oder Fig. 8a gezeigten Ausführungsformen eingesetzt werden kann.
Fig. 12a zeigt das System aus Inhalator und Kapsel (71) im zusammengesetzten Zustand, der hier auch dem Transportzustand entspricht. Die (nicht gezeigten) vorgefertigten Löcher (72a, 72b) der Kapsel (71) werden durch die Innenwand einer schalenförmig gestalteten Kapselaufnahme (173a) verschlossen. Die Kapselaufnahme (173a) ist Bestandteil eines Zugelements (173). Für Kapseln (71), von denen ein Kapselelement z.B. die Kapselkappe (1) einen größeren Außendurchmesser als der zugängliche Teil des anderen Kapselelementes z.B. des Kapselkörpers (2) hat, weist die Kapselaufnahme (173a) vorzugsweise eine entsprechende Veränderung des Innendurchmessers auf: das Kapselelement mit geringerem Durchmesser befindet sich an der Kapselaufnahme optional, wie hier in Fig. 12 gezeigt, weiter in Zugrichtung des Zugelements (173) als das Kapselelement mit größerem Durchmesser. Der innere Durchmesser der Kapselaufnahme (173a) ist entsprechend an die äußere Gestalt der Kapsel (71) angepasst, so dass auch er in gleicher Weise wie die Kapsel (71) schrittweise breiter wird. Im Transportzustand ist die Kapsel (71) somit ohne nennenswerten Bewegungsspielraum im System eingeschlossen.

Vorzugsweise besteht das Zugelement (173) aus einem teilweise flexiblen und/oder formbaren Material, wie besonders bevorzugt aus einer Blisterfolie, die in einem Tiefziehprozess strukturiert wurde.
Bevorzugt besteht das Zugelement (173) (wie in Fig. 12 abgebildet) aus zwei Hälften, die vorzugsweise bereits über eine Scharnier-ähnliche Stelle wie ein Filmscharnier (173b) miteinander verbunden sind (d.h. die beiden Hälften werden vorzugsweise als ein Teil bzw. in den gleichen Arbeitsschritten gefertigt). Weiterhin bevorzugt besteht das Zugelement (173) aus einem Tiefziehteil und/oder wird in einem Tiefziehprozess vorzugsweise unter Wärmeeinwirkung aus einer Tiefziehfolie bzw. Blisterfolie hergestellt, d.h. aus einer Folie wie sie in der Medizintechnik allgemein üblich für Blisterverpackungen beispielsweise von Tabletten verwendet wird. Diese Blisterfolien bestehen vorzugsweise aus mehreren Lagen und beinhalten bevorzugt Polyethylen und/oder Aluminium. Besonders bevorzugt werden Folien mit Dicken kleiner 1 Millimeter, besonders bevorzugt mit Dicken im Bereich von 50 bis 200 Mikrometer wie beispielsweise 60, 80 oder 120 Mikrometer eingesetzt. Die beiden Hälften werden vorzugsweise in einem Siegelprozess am Zugende des Zugelements (173) und zwar in einem Bereich zwischen dem Filmscharnier (173b) und der Kapselaufnahme (173a) verbunden. Dies resultiert in einer geeigneten Steifigkeit des Zugelements (173) und seiner Griffhilfe (173e). Für eine solche Verbindung ist die verwendete Blisterfolien bevorzugt mit einer Klebeschicht oder einem Siegellack beschichtet. Im direkten Bereich der Kapselaufnahme (173a) werden die Hälften nicht miteinander verbunden, so dass ein zum Kapselende des Zugelements (173) offener Schlitz (173d) bestehen bleibt. Dieser Schlitz (173d) erstreckt sich über die Kapselaufnahme (173a) hinaus in Richtung Mitte des Zugelements (173), so dass dort ein Hohlraum bestehen bleibt, in den ein Stab (75) des Inhalators eintauchen kann. Bevorzugt ist das Zugelement (173) im Bereich der Kapselaufnahme (173a) so ausgelegt, dass in diesem Bereich die Hälften sozusagen selbstschließend angeordnet sind, d.h. die Hälften die Kapsel einklemmen und gut in Position halten und dabei gewissermaßen unter einer Federvorspannung stehen. Durch die damit verbundene Federelastizität können die Hälften bei Druck auf die Kapsel (71) entlang der Längsachse des Zugelements (173) sich voneinander weg biegen und die Kapsel (71) freigeben.

Im Montageverfahren kann beispielsweise eine vorgelochte und befüllte Kapsel (71) in eine Hälfte der Kapselaufnahme (173a) eingelegt werden, worauf das Zugelement (173) zusammengeklappt wird und die zweite Hälfte der Kapselaufnahme (173a) ebenfalls die Kapsel (71) bedeckt. Nach dem Zusammenklappen bleibt ein Schlitz (173d) am Zugelement (173) bestehen (der wie oben beschrieben insbesondere im Bereich der Griffhilfe (173e) durch Siegelung geschlossen wird). Die Kapsel (71) sollte daher orientiert in die Kapselaufnahme (173a) eingesetzt werden, so dass die Löcher (72) tatsächlich von den Schalen der Kapselaufnahme (173a) voll abgedeckt werden. Bevorzugt befinden sich vorgefertigten Löcher (72) an der Kapsel (71) nur an einer Längsseite der Kapsel (71) (bzw. weniger als 180 Grad über den Umfang der Kapsel (71) verteilt). Dadurch kann die vorbefüllte Kapsel (71) beim orientierten Einsetzen in die erste Hälfte der Kapselaufnahme (173a) - nämlich mit der Orientierung, dass die Löcher (72) bezogen auf die Schwerkraft nach oben und vorzugsweise in die zweite Hälfte der Kapselaufnahme (173a) hinein zeigen - ohne Pulververlust durch die Löcher montiert werden.
Ein im Prozess vorangehendes Befüllverfahren kann wie folgt ablaufen: Die Kapsel (71) wird in einem formschlüssigen Kapselträger in einer Abfüllanlage befüllt, wobei der Kapselträger zunächst den Kapselkörper (2) aufnimmt. Dieser Kapselträger hält den Kapselkörper (2) während des Füllprozesses und verschließt beim Befüllen die Löcher (72) im Kapselkörper (2). Nach dem Schließen der Kapsel (71) durch Aufschieben der Kapselkappe (1) wird der Kapselträger um 90° gedreht (wobei die Löcher nach oben, d.h. in entgegengesetzter Richtung zur Schwerkraft zeigen) und die Kapsel (71) wird vom Kapselträger in das Zugelement (173) eingeschoben oder alternativ in das noch offene Zugelement eingelegt, welches dann zugeklappt und dann verschlossen bzw. versiegelt wird. Optional wird der Verschluss der Löcher (72) in der Kapsel (71) durch die Schalen der Kapselaufnahme (173a) durch einen Pressschritt verbessert.

Alternativ zum Einlegen der befüllten und vorgelochten Kapsel (71) in eine Hälfte der Kapselaufnahme (173a) ist bei geeigneter Flexibilität des Materials des Zugelements (173) auch ein Verfahren analog Fig. 10 für die Befüllung und das Einsetzen einer vorgelochten Kapsel (71) anwendbar: Die beiden Hälften des Zugelements (173) werden zusammengeklappt und im Bereich der Kapselaufnahme aufgebogen bzw. aufgeklappt (oder das Zugelement wird erst im Bereich der Kapselaufnahme (173a) aufgebogen und anschließend zusammengeklappt), so dass ein Kapselelement einsetzt und darin befüllt werden kann. Anschließend wird das zweite Kapselement auf das erste aufgeschoben und die Kapselaufnahmen werden wieder zurückgebogen.
Alternativ kann die Kapsel (71) in einem formschlüssigen Kapselträger in einer Abfüllanlage befüllt werden. Dieser Kapselträger verschließt beim Befüllen die Löcher im Kapselkörper (2). Nach dem Schließen der Kapsel (71) durch die Kapselkappe (2), kann dann die Kapsel (71) direkt in das senkrecht darüber befindliche Zugelement (173) geschoben werden (sozusagen von unten bezogen auf die Wirkrichtung der Schwerkraft).

Wiederrum ein alternatives Verfahren beinhaltet den Einsatz einer Kapsel (1) mit zwei verschiedenen Einsteckstellungen, wie sie beispielsweise anhand der Figuren 1 bis 5 beschrieben wird. Hierbei wird ein Kapselelement befüllt und mit dem anderen Kapselelement so zusammen gesteckt, dass die Löcher (6,7) der beiden Kapselelemente nicht übereinander liegen, die Kapsel (1) also komplett verschlossen ist (erste Einsteckstellung). Die Kapsel (1) wird beispielsweise in eine Hälfte der Kapselaufnahme (173a) eingelegt, und beim Zusammenklappen des Zugelements (173) durch die zweite Hälfte der Kapselaufnahme (173a) abgedeckt. Anschließend wird von einer Seite beispielsweise beidseitig von außen auf die Kapselaufnahme (173) gedrückt, so dass an der innen liegenden Kapsel (1) die beiden Kapselelemente weiter ineinander gleiten und die Löcher (6,7) an den beiden Kapselelementen in Überdeckung gelangen (die zweite Einsteckstellung wird erzielt). Gleichzeitig wird die Kapselaufnahme (173a) so verformt, dass sie sich sich an die nun kürzere Kapsel (1) anschmiegt.

Bevorzugt besteht der Körper (100) des Inhalators (wobei Kapselkammer (74) vom Körper (100) gebildet werden) aus zwei Hälften, besonders bevorzugt aus zwei Tiefziehteilen, die Körper (100) entlang einer Längsachse in zwei Hälften teilen (z.B. kann man Fig. 12 a anstelle eines schematischen Längsschnitts auch so interpretieren, dass sie ein den Körper (100) bildendes Tiefziehteil zeigt, in welches das Zugelement (173) mit Kapsel (71) eingesetzt ist; das eigentlich dünne Tiefziehteil ist in der Darstellungsebene zu einem Pfalz umgebogen, der als Verbindungsfläche dient). Bevorzugt werden die beiden Hälften aus Folien insbesondere Tiefziehfolien oder Blisterfolien gefertigt, wie sie beim Zugelement (173) wie oben beschrieben bevorzugt verwendet werden. Somit können Zugelement (173) und Körper (100) aus dem gleichen Material gefertigt werden.
Des Weiteren bevorzugt umfasst der Körper (100) auch den Bereich des Mundstücks (78), auf den die Lippen des Anwenders aufgelegt werden; dieser Lippenbereich des Mundstücks (78) kann aber auch durch ein zusätzliches Bauteil gebildet werden, dass mit dem Körper (100) verbunden wird.

Im Montageverfahren wird dann beispielsweise das Zugelement (173) mit Kapsel (71) in eine beispielsweise von einem Tiefziehteil gebildete Hälfte des Körpers (100) derart eingelegt, dass sich die Kapselaufnahme (173a) mit der Kapsel (71) in der Kapselkammer (74) befindet und der Stab (75) zwischen Griffhilfe (173e) und Kapselaufnahme (173a) in den Schlitz (173d) am Zugelement eintaucht. Anschließend wird die zweite Hälfte des Körpers (100), also beispielsweise das zweite Tiefziehteil, (vorzugsweise spiegelbildlich) auf die erste Hälfte aufgelegt und/oder gesteckt. Um eine Verbindung beider Hälften ohne Spalt zwischen den Hälften sicherzustellen, werden die Hälften bzw. die beiden Tiefziehteile miteinander verbunden, beispielsweise durch Schweißen, Kleben, Laminieren oder bevorzugt durch Verbinden in einem Siegelprozess. Beim Siegeln kann ein in der Herstellung von Blister üblicher Siegelprozess angewendet werden. Ein möglicher Fertigungsprozess kann z.B. das Aufbringen einer Siegelaktiveschicht (Siegellack) auf die Hälften des Körpers (100) direkt nach dem Tiefziehprozess der beinhalten. Die Versieglung mittels Druck und Wärme erfolgt dann erst später bei der Montage der Teile.
Bei der Verwendung von Tiefziehteilen als Hälften des Körpers (100), können die Hälften beispielsweise aus einer Tiefziehfolie abgeform sein, die im Bereich der Verbindungsstelle als Pfalz ausgeformt ist, so dass an den ansonsten dünnwandigen Bauteilen eine Auflagefläche für die eine Siegelung oder Schweißung etc. bestehen bleibt. Alternativ können die beiden Hälften auch als dickwandigere Teile ohne Pfalz in einem Kunststoff-Spritzgussverfahren hergestellt und später miteinander vorzugsweise in einem Ultraschallschweißverfahren verschweißt werden.

Zur Anwendung des Inhalators wird dann (wie in Fig. 12b gezeigt) die Kapselaufnahme (173a) aus dem Mundstück (78) herausgezogen. Die Schlitze (173d) an Kapselaufnahme (173a) und Zugelement (173) insgesamt ermöglichen hierbei das Herausziehen der Kapselaufnahme (173a) am Stab (75) vorbei (analog der Funktion des Schlitz (73d) in Fig. 10). Der in das Mundstück (78) eingesetzte und sich quer durch die Mundstücköffnung erstreckende Stab (75) bewirkt, dass die Kapsel (71) in der Kapselkammer (74) im Mundstück (78) verbleibt und nicht mit der Kapselaufnahme (173a) wieder herausgezogen werden kann. In einer Ausführungsform, in welcher der Körper (100) des Inhalators aus zwei Hälften zusammengesetzt ist, ist auch der Stab bevorzugt derart zweigeteilt, dass je eine Hälfte des Stabs (75) von einer Hälfte des Körpers (100) gebildet wird. Somit ist der Stab (75) ein integrierter Bestandteil des Körpers (100) und ist bevorzugt kein Einzelteil, so dass keine zusätzliche Montage des Stabs (75) notwendig ist. Beim Verschweißen oder Versiegeln der beiden Hälften des Körpers (100) werden bevorzugt auch die beiden Hälften des Stabs (75) miteinander verbunden.
Um dem Anwender das Herausziehen des Zugelements (173) mit Kapselaufnahme (173a) aus dem Mundstück (78) zu erleichtern, weist das Zugelement (173) vorzugsweise eine Griffhilfe (173e) auf. Diese Griffhilfe (173e) kann beispielsweise wie in Fig. 12b gezeigt durch eine flache Lasche bevorzugt mit Öffnung bzw. Griffloch gebildet werden. Diese Lasche befindet sich vorzugsweise mittig oben am Zugelement (173) und ragt im zusammengesetzten Zustand des Systems (Fig. 12a) an der Seite des Mundstücks (78) aus dem Inhalator heraus, so dass der Anwender zur Aktivierung des Systems lediglich das Zugelement (173) an der Griffhilfe (173e) oder Lasche aus dem Mundstück (78) ziehen muss.
Bevorzugt werden die hier beschriebenen Systeme mit einer medizinischen Formulierungen betrieben, die ein Bestandteil aufweist, das in der Offenbarung der europäischen Patentanmeldung mit der Anmeldenummer 12151105.9 auf Seite 26 Zeile 12 bis Seite 63 Zeile 2 genannt wird, oder die einer der dort genannten Formulierungen entspricht.

### Bezugszeichenliste

- 1: Kapselkappe
- 2: Kapselkörper
- 3: Sicke (an Kapselkappe)
- 4: Sicke (an Kapselkappe)
- 5: Wulst (an Kapselkörper)
- 6 , 6a, 6b: Loch (an Kapselkappe)
- 7 , 7a, 7b: Loch (an Kapselkörper)
- 8: Steg (an Kapselkörper)
- 9: Nut (an Kapselkappe)
- 11: zusammengesetzte Kapsel (geeignet für Lochabdeckung mit Ring)
- 22: Ring
- 22a: Wulst (an Ring)

- 40: Pulver
- 42a: Loch (seitlich unten an geschlossener Kapsel)
- 42b: Loch (seitlich oben an geschlossener Kapsel)
- 70: Gerät
- 71: Kapsel (geschlossen)
- 71a,b: halbkugelförmige Enden (der geschlossenen Kapsel)
- 72, 72a, 72b: vorgefertigte Löcher
- 73: Röhre
- 73a: Kragen (an Röhre)
- 73b: Sicke (außen am Kragen der Röhre)
- 73d: Schlitz (an Röhre)
- 73e: Griffhilfe (an Röhre)
- 73f: Federarm (an Röhre)
- 73g: Abstand (zwischen Röhren)
- 73k: Abschlussbereich
- 73s: Ausnehmung (in Wand der Röhre)
- 74: Kapselkammer
- 75: Stab
- 76: Einlass
- 77: Schieber
- 77a: Verjüngung (am Schieber)
- 77b: Federarm (am Schieber)
- 78: Mundstück
- 78b: Wulst (am unteren Innenrand des Mundstücks)
- 78c: Grifffläche (am Mundstück)
- 78d: Mundrohr (im Mundstück)
- 78e: Mittelwand (im Mundstück)
- 91: Pfeil (in Strömungsrichtung der Luft am Einlass)
- 92: Pfeil (in Einatmungsrichtung)
- 100: Körper (des Inhalators)
- 173: Zugelement
- 173a: Kapselaufnahme
- 173b: Filmscharnier
- 173d: Schlitz (Zugelement)
- 173e: Griffhilfe (an Zugelement)

## Patentansprüche

1. System aus einem Inhalator und einer Kapsel zur Verwendung als Reservoir für eine pharmazeutische, vorzugsweise pulverförmige Zubereitung im Inhalator wobei
- die Kapsel zwei einseitig offene Kapselelemente, nämlich einen Kapselkörper (2) und eine Kapselkappe (1), umfasst, die teleskopartig über ihre Öffnungen ineinander gesteckt werden können, so dass ein Hohlraum gebildet wird, und
- der Inhalator eine Kapselkammer (74) aufweist,
**dadurch gekennzeichnet, dass**
- Kapselkörper (2) und/oder Kapselkappe (1) zusätzlich zur einseitigen Öffnung mindestens je ein vorgefertigtes Loch (7, 6) aufweisen,
- die Kapsel (71) vor Anwendung des Inhalators in einer Kapselaufnahme (173a) bevorratet ist, wobei die Kapselaufnahme (173a) mindestens ein Loch (72a, 72b) abdichtet, das in den Hohlraum der Kapsel (71) führt, und/oder alle Löcher (72a, 72b) abdichtet, die in den Hohlraum der Kapsel (71) führen, und
- die Kapselaufnahme (173a) zumindestens teilweise in der Kapselkammer (74) angeordnet ist, und die Kapselaufnahme (173a) durch einen Zugmechanismus derart aus der Kapselkammer (74) entfernbar ist, dass die Kapsel (71) bei Entfernung der Kapselaufnahme (173a) in der Kapselkammer (74) zurückbleibt.

2. System nach Anspruch 1, **dadurch gekennzeichnet, dass** die Kapselaufnahme (173a) Teil eines Zugelements (173) ist oder von einem Zugelement (173) gebildet wird.

3. System nach einem der vorherigen Ansprüche, **dadurch gekennzeichnet, dass** die Kapselelemente aus einem Kunststoffmaterial, vorzugsweise beide aus dem gleichen Material und insbesondere aus Polyethylen, Polycarbonat, Polyester, Polypropylen oder Polyethylenterrephthalat, in einem Spritzgussverfahren gefertigt sind und die vorgefertigten Löcher (6, 7) bereits im gleichen Spritzgussverfahren ausgebildet worden sind.

4. System nach einem der vorherigen Ansprüche **dadurch gekennzeichnet, dass** der Inhalator ein Mundstück (78) mit einem Mundrohr (78d) aufweist, das die Kapselkammer (74) mit einem Luftauslass verbindet, wobei der Luftauslass eine Öffnung vorzugsweise mittig an dem bei Anwendung mundseitigen Ende des Mundstücks (78) darstellt,
und dass sich ein Bauteil, das die Kapselaufnahme (173a) bildet oder umfasst, durch das Mundrohr (78d) im Inhalator erstreckt.

5. System nach einem der vorherigen Ansprüche **dadurch gekennzeichnet, dass** der Inhalator einen Steg, Stift oder Stab (75) aufweist, der die Kapselkammer (74) begrenzt und sich durch das Bauteil erstreckt, das die Kapselaufnahme (173a) bildet, und dass besagtes Bauteil eine Aussparung aufweist, durch die der Steg, Stift oder Stab (75) bei einer Relativbewegung zwischen Bauteil und Kapselkammer (74) gleiten kann.

6. System nach einem der vorherigen Ansprüche **dadurch gekennzeichnet, dass** der Inhalator ein Mundstück (78) mit einem bei Anwendung mundseitigen Ende aufweist und sich mittig an diesem mundseitigen Ende eine Öffnung befindet, die mit der Kapselkammer (74) verbunden ist, und dass das Bauteil, das die Kapselaufnahme (173a) bildet, als Kappe ausgebildet ist, die in einem Transportzustand des Systems die Öffnung am mundseitigen Ende verschließt, vorzugsweise so dass dabei die Bereiche, an denen ein Anwender bei Anwendung des Inhalators seine Lippen ansetzt, abgedeckt werden.

7. System nach einem der vorherigen Ansprüche **dadurch gekennzeichnet, dass** das Bauteil, das die Kapselaufnahme (173a) bildet, Elemente wie insbesondere Federarme aufweist, die verhindern, dass die Kapselaufnahme (173a) nach ihrer vorzugsweise vollständigen Entfernung aus der Kapselkammer (74) wieder zerstörungsfrei in die Kapselkammer (74) eingesetzt werden kann.

8. System nach einem der vorangegangenen Ansprüche **dadurch gekennzeichnet, dass** die Kapselaufnahme (173a) durch eine Tiefziehfolie oder Blisterfolie gebildet wird.

9. System nach einem der vorangegangenen Ansprüche **dadurch gekennzeichnet, dass** der Inhalator einen eine Kapselkammer (74) bildenden Körper (100) aufweist, der durch zwei vorzugsweise als Hälften gestaltete Teile gebildet wird, die durch eine mittels Schweißen, Laminieren, Kleben oder Siegeln entstandene Verbindung mit einander verbunden sind.

10. System nach einem der vorangegangenen Ansprüche **dadurch gekennzeichnet, dass** der Inhalator einen eine Kapselkammer (74) bildenden Körper (100) aufweist, der im Wesentlichen aus einer Tiefziehfolie oder Blisterfolie besteht.

11. System nach einem der Ansprüche 1 bis 10 **dadurch gekennzeichnet, dass**
- sich die Kapsel in einem Transportzustand des Systems im Inhalator befindet,
- die zusammengesetzte Kapsel (71) mindestens ein vorgefertigtes Loch (72, 72a, 72b) aufweist,
- die Kapselaufnahme (173a) eine vorzugsweise dehnbaren und/oder biegsamen Folie umfasst oder von dieser gebildet wird, wobei diese Folie die Kapsel derart zumindest teilweise umschließt, dass die Folie das mindestens eine vorgefertigte Loch (72, 72a, 72b) und/oder alle Löcher der Kapsel (71) im Transportzustand des Systems verschließt,
- ein Teil der Folie an einem Ende der Kapsel (71) über die Kapsel (71) hinausragt und/oder die Folie mit einem Zugband verbunden ist,
- der Inhalator eine Öffnung aufweist oder eine Öffnung am Inhalator freigelegt werden kann, und die Folie an ihrem überstehenden Teil und/oder an ihrem Zugband durch die Öffnung aus dem Inhalator herausgezogen werden kann, wobei das mindestens eine vorgefertigte Loch (72, 72a, 72b) und/oder alle Löcher an der Kapsel (71) frei gelegt werden.

12. System aus Inhalator und Kapsel nach einem der vorangegangenen Ansprüche **dadurch gekennzeichnet, dass** zwei Kapseln (71) zum System gehören und der Inhalator zwei Kapselkammern (74) und zwei Kapselaufnahme (173a)n aufweist, wobei vorzugsweise die beiden Kapseln mit verschiedenen Formulierungen und/oder verschiedenen Formulierungsmengen befüllt sind.

13. Verfahren zur Bereitstellung eines Systems aus Inhalator und Kapsel nach einem der vorangegangenen Ansprüche **dadurch gekennzeichnet, dass** eine Kapselaufnahme (173a), die Teil eines Zugelement (173)s ist oder von einem Zugelement (173) gebildet wird, mit darin enthaltener Pulver-befüllter und vorgelochter Kapsel (71), mit den anderen Bauteilen des Inhalators zusammengesetzt wird, wobei der die Kapsel (71) aufnehmende Teil der Kapselaufnahme (173a) in einer Kapselkammer (74) des Inhalators angeordnet wird, und, dass nach Zusammensetzen der Bauteile des Inhalators das System in einem vorzugsweise für Transport oder Lagerung geeigneten Zustand vorliegt, in dem die Löcher der von Kapselkörper (2) und Kapselkappe (1) gebildeten Kapsel (71) verschlossen sind, und dass das System durch Ziehen am Zugelement (173) in einen anwendungsbereiten Zustand überführt wird, wobei durch dieses Ziehen die Löcher der Kapsel freigegeben werden.

14. Verfahren nach Anspruch 13 **dadurch gekennzeichnet, dass** der Inhalator einen die Kapselkammer (74) bildenden Körper (100) aufweist, der durch zwei vorzugsweise als Hälften gestaltete Teile gebildet wird, wobei die Kapselaufnahme (173a) mit der Kapsel (71) zunächst an die Stelle der Kapselkammer (74) in den ersten Teil des Körpers (100) eingelegt wird, dann die Kapselkammer (74) durch Auflegen des zweiten Teils des Körpers (100) vervollständigt wird und die beiden Teile des Körpers (100) durch Schweißen, Laminieren, Kleben oder Siegeln mit einander verbunden werden.

15. Verfahren nach Anspruch 13 oder 14 **dadurch gekennzeichnet, dass** vor dem Zusammensetzen der Kapselaufnahme (173a) mit anderen Bauteilen des Inhalators zunächst ein einseitig offenes Kapselelement - der Kapselkörper (2) - in eine Kapselaufnahme (173a) eingesetzt wird, der Kapselkörper (2) mit einer abgemessenen Menge vorzugsweise pulverförmiger pharmazeutischer Zubereitung befüllt wird, der Kapselkörper anschließend mit einer Kapselkappe (1) verschlossen wird.

## Claims

1. System composed of an inhaler and a capsule for use as a reservoir for a pharmaceutical, preferably a powdered preparation in the inhaler, wherein
- the capsule comprises two capsule elements open at one end, namely a capsule body (2) and a capsule cap (1), which can be fitted into one another telescopically through their openings, so as to form a cavity, and
- the inhaler comprises a capsule chamber (74),
**characterized in that**
- the capsule body (2) and/or the capsule cap (1) comprise in addition to the opening at one end at least one prefabricated hole (7, 6) in each case,
- before the inhaler is used, the capsule (71) is stored in a capsule receptacle (173a), the capsule receptacle (173a) sealing off at least one hole (72a, 72b) which leads into the cavity of the capsule (71), and/or sealing off all the holes (72a, 72b) that lead into the cavity of the capsule (71), and
- the capsule receptacle (173a) is at least partly arranged in the capsule chamber (74), and the capsule receptacle (173a) can be removed from the capsule chamber (74) by means of a pulling mechanism such that when the capsule receptacle (173a) is removed the capsule (71) remains in the capsule chamber (74).

2. System according to claim 1, **characterized in that** the capsule receptacle (173a) is part of a pulling element (173) or is formed by a pulling element (173).

3. System according to either one of the preceding claims, **characterized in that** the capsule elements are made from a plastics material, preferably both from the same material and, in particular, from polyethylene, polycarbonate, polyester, polypropylene or polyethylene terephthalate, in an injection molding process and the prefabricated holes (6, 7) have already been formed in the same injection molding process.

4. System according to any one of the preceding claims, **characterized in that** the inhaler comprises a mouthpiece (78) with a mouth tube (78d) that connects the capsule chamber (74) to an air outlet, the air outlet representing an opening preferably in the center of the end of the mouthpiece (78) that is next to the mouth during use, and **in that** a component that forms or encompasses the capsule receptacle (173a) extends through the mouth tube (78d) in the inhaler.

5. System according to any one of the preceding claims, **characterized in that** the inhaler comprises a tongue, pin or bar (75) that delimits the capsule chamber (74) and extends through the component that forms the capsule receptacle (173a), and **in that** said component comprises a recess through which the tongue, pin or bar (75) can slide during relative movement between the component and the capsule chamber (74).

6. System according to any one of the preceding claims, **characterized in that** the inhaler comprises a mouthpiece (78) with one end that is closest to the mouth during use, and forms an opening that is connected to the capsule chamber (74) in the middle of said mouth end, and **in that** the component that forms the capsule receptacle (173a) is embodied as a cap which, in a transporting state of the system, closes off the opening at the mouth end, preferably in such a way that the areas to a which a user places his lips when using the inhaler are covered.

7. System according to any one of the preceding claims, **characterized in that** the component that forms the capsule receptacle (173a) comprises elements such as spring arms, in particular, which prevent the capsule receptacle (173a) from being non-destructively reinserted in the capsule chamber (74) after being preferably fully removed from the capsule chamber (74).

8. System according to any one of the preceding claims, **characterized in that** the capsule receptacle (173a) is formed by a thermoformed film or blister film.

9. System according to any one of the preceding claims, **characterized in that** the inhaler comprises a body (100) forming a capsule chamber (74), which is formed by two parts, preferably in the form of halves, which are joined together by a joint formed by welding, laminating, gluing or sealing.

10. System according to any one of the preceding claims, **characterized in that** the inhaler comprises a body (100) forming a capsule chamber (74) which consists essentially of a thermoformed film or blister film.

11. System according to any one of claims 1 to 10, **characterized in that**
- the capsule is located in the inhaler in a transporting state of the system,
- the assembled capsule (71) comprises at least one prefabricated hole (72, 72a, 72b),
- the capsule receptacle (173a) comprises or is formed by a preferably extensible and/or flexible film, said film at least partially surrounding the capsule such that, in the transporting state of the system, the film closes off the at least one prefabricated hole (72, 72a, 72b) and/or all the holes in the capsule (71),
- part of the film protrudes beyond the capsule (71) at one end of the capsule (71) and/or the film is connected to a pull strip,
- the inhaler comprises an opening or an opening can be exposed on the inhaler, and the film can be pulled out of the inhaler by its protruding part and/or by its pull strip through the opening, whereupon the at least one prefabricated hole (72, 72a, 72b) and/or all the holes on the capsule (71) are exposed.

12. System comprising an inhaler and capsule according to any one of the preceding claims, **characterized in that** two capsules (71) belong to the system and the inhaler comprises two capsule chambers (74) and two capsule receptacles (173a), the two capsules preferably being filled with different formulations and/or different amounts of formulation.

13. Method for providing a system comprising an inhaler and capsule according to any one of the preceding claims, **characterized in that** a capsule receptacle (173a) which is part of a pulling element (173) or is formed by a pulling element (173) with a powder-filled, pre-perforated capsule (71) contained therein, is assembled with the other components of the inhaler, the part of the capsule receptacle (173a) that holds the capsule (71) being arranged in a capsule chamber (74) of the inhaler, and **in that**, after the assembly of the components of the inhaler, the system is in a state that is preferably suitable for transportation or storage, in which the holes of the capsule (71) formed by the capsule body (2) and the capsule cap (1) are closed off, and **in that** the system is converted into a state ready for use by pulling the pulling element (173), said pulling action exposing the holes in the capsule.

14. Method according to claim 13, **characterized in that** the inhaler comprises a body (100) forming the capsule chamber (74), which is formed by two parts preferably in the form of halves, the capsule receptacle (173a) with the capsule (71) initially being placed in the first part of the body (100) in place of the capsule chamber (74), then the capsule chamber (74) being completed by the placement of the second part of the body (100), and the two parts of the body (100) being joined together by welding, laminating, gluing or sealing.

15. Method according to either claim 13 or claim 14, **characterized in that**, before the capsule receptacle (173a) is assembled with other components of the inhaler, first of all a capsule element - the capsule body (2) - open at one end is inserted in a capsule receptacle (173a), the capsule body (2) is filled with a measured amount of preferably powdered pharmaceutical preparation and the capsule body is then closed off with a capsule cap (1).

## Revendications

1. Système composé d'un inhalateur et d'une capsule pour l'utilisation en tant que réservoir pour une préparation pharmaceutique, de préférence poudreuse, dans l'inhalateur, dans lequel
- la capsule comprend deux éléments de capsule ouverts d'un côté, à savoir un corps de capsule (2) et un capuchon de capsule (1), qui peuvent être enfichés l'un dans l'autre de manière télescopique par le biais de leurs ouvertures de sorte qu'une cavité soit formée, et
- l'inhalateur présente une chambre de capsule (74), **caractérisé en ce que**
- le corps de capsule (2) et/ou le capuchon de capsule (1) présente outre l'ouverture unilatérale au moins chacun un trou préfabriqué (7, 6),
- la capsule (71) est stockée, avant l'emploi de l'inhalateur, dans un logement de capsule (173a), dans lequel le logement de capsule (173a) rend étanche au moins un trou (72a, 72b) qui mène dans la cavité de la capsule (71), et/ou rend étanche tous les trous (72a, 72b) qui mènent dans la cavité de la capsule (71), et
- le logement de capsule (173a) est disposé au moins partiellement dans la chambre de capsule (74), et le logement de capsule (173a) peut être retiré par un mécanisme de traction de la chambre de capsule (74) de telle manière que la capsule (71) reste, lors du retrait du logement de capsule (173a), dans la chambre de capsule (74).

2. Système selon la revendication 1, **caractérisé en ce que** le logement de capsule (173a) fait partie d'un élément de traction (173) ou est formé par un élément de traction (173).

3. Système selon l'une quelconque des revendications précédentes, **caractérisé en ce que** les éléments de capsule sont fabriqués en un matériau plastique, de préférence les deux en le même matériau, et en particulier en polyéthylène, polycarbonate, polyester, polypropylène ou téréphtalate de polyéthylène, dans un procédé de moulage par injection et les trous préfabriqués (6, 7) ont déjà été réalisés dans le même procédé de moulage par injection.

4. Système selon l'une quelconque des revendications précédentes, **caractérisé en ce que** l'inhalateur présente un embout (78) buccal avec un tube buccal (78d) qui raccorde la chambre de capsule (74) à une sortie d'air, dans lequel la sortie d'air constitue une ouverture, de préférence au milieu à l'extrémité côté bouche de l'embout (78) buccal lors de l'emploi, et qu'un composant, qui forme ou comprend le logement de capsule (173a), s'étend au travers du tube buccal (78d) dans l'inhalateur.

5. Système selon l'une quelconque des revendications précédentes, **caractérisé en ce que** l'inhalateur présente une nervure, une tige ou une barre (75) qui délimite la chambre de capsule (74) et s'étend au travers du composant qui forme le logement de capsule (173a), et que ledit composant présente un évidement par lequel la nervure, la tige ou la barre (75) peut glisser en cas de mouvement relatif entre le composant et la chambre de capsule (74).

6. Système selon l'une quelconque des revendications précédentes, **caractérisé en ce que** l'inhalateur présente un embout (78) buccal avec une extrémité côté bouche lors de l'emploi et une ouverture se trouve au milieu à cette extrémité côté bouche qui est raccordée à la chambre de capsule (74), et que le composant, qui forme le logement de capsule (173a), est réalisé en tant que capuchon qui ferme, dans un état de transport du système, l'ouverture à l'extrémité côté bouche, de préférence de sorte que les zones au niveau desquelles un utilisateur pose ses lèvres lors de l'emploi de l'inhalateur soient recouvertes.

7. Système selon l'une quelconque des revendications précédentes, **caractérisé en ce que** le composant, qui forme le logement de capsule (173a), présente des éléments tels qu'en particulier des bras de ressort qui empêchent que le logement de capsule (173a) ne puisse être de nouveau utilisé après son retrait, de préférence complet, de la chambre de capsule (74) sans destruction dans la chambre de capsule (74).

8. Système selon l'une quelconque des revendications précédentes, **caractérisé en ce que** le logement de capsule (173a) est formé par un film à emboutissage profond ou film blister.

9. Système selon l'une quelconque des revendications précédentes, **caractérisé en ce que** l'inhalateur présente un corps (100) formant une chambre de capsule (74) qui est formé par deux parties, conçues de préférence en tant que moitiés, qui sont raccordées par un raccordement résultant par soudage, laminage, collage ou scellage l'une avec l'autre.

10. Système selon l'une quelconque des revendications précédentes, **caractérisé en ce que** l'inhalateur présente un corps (100) formant une chambre de capsule (74) qui se compose sensiblement d'un film à emboutissage profond ou d'un film blister.

11. Système selon l'une quelconque des revendications 1 à 10, **caractérisé en ce que**
- la capsule se trouve, dans un état de transport du système, dans l'inhalateur,
- la capsule (71) assemblée présente au moins un trou (72, 72a, 72b) préfabriqué,
- le logement de capsule (173a) comprend un film, de préférence extensible et/ou flexible, ou est formé par celui-ci, dans lequel ce film entoure au moins partiellement la capsule de telle manière que le film ferme l'au moins un trou (72, 72a, 72b) préfabriqué et/ou tous les trous de la capsule (71) dans l'état de transport du système,
- une partie du film dépasse de la capsule (71) à une extrémité de la capsule (71) et/ou le film est raccordé à une bande de traction,
- l'inhalateur présente une ouverture ou une ouverture peut être libérée au niveau de l'inhalateur, et le film peut être retiré de l'inhalateur, au niveau de sa partie en saillie et/ou au niveau de sa bande de traction, au travers de l'ouverture, dans lequel l'au moins un trou (72, 72a, 72b) préfabriqué et/ou tous les trous sont libérés au niveau de la capsule (71).

12. Système composé d'un inhalateur et d'une capsule selon l'une quelconque des revendications précédentes, **caractérisé en ce que** deux capsules (71) appartiennent au système et l'inhalateur présente deux chambres de capsule (74) et deux logements de capsule (173a), dans lequel de préférence les deux capsules sont remplies de différentes formulations et/ou différentes quantités de formulation.

13. Procédé de fourniture d'un système composé d'un inhalateur et d'une capsule selon l'une quelconque des revendications précédentes, **caractérisé en ce qu'**un logement de capsule (173a), qui fait partie d'un élément de traction (173) ou est formé par un élément de traction (173), avec une capsule (71) prétrouée et remplie de poudre contenue dedans, est assemblé avec les autres composants de l'inhalateur, dans lequel la partie recevant la capsule (71) du logement de capsule (173a) est disposée dans une chambre de capsule (74) de l'inhalateur et que, après l'assemblage des composants de l'inhalateur, le système se présente dans un état, approprié de préférence au transport ou stockage, dans lequel les trous de la capsule (71) formée par le corps de capsule (2) et le capuchon de capsule (1) sont fermés, et que le système est transféré par traction sur l'élément de traction (173) dans un état prêt à l'emploi, dans lequel les trous de la capsule sont libérés par cette traction.

14. Procédé selon la revendication 13, **caractérisé en ce que** l'inhalateur présente un corps (100) formant la chambre de capsule (74) qui est formé par deux parties conçues de préférence en tant que moitiés, dans lequel le logement de capsule (173a) est introduit avec la capsule (71) tout d'abord à l'endroit de la chambre de capsule (74) dans la première partie du corps (100), puis la chambre de capsule (74) est complétée par la pose de la seconde partie du corps (100) et les deux parties du corps (100) sont raccordées l'une avec l'autre par soudage, laminage, collage ou scellage.

15. Procédé selon la revendication 13 ou 14, **caractérisé en ce que**, avant l'assemblage du logement de capsule (173a) avec d'autres composants de l'inhalateur, tout d'abord un élément de capsule ouvert sur un côté - le corps de capsule (2) - est inséré dans un logement de capsule (173a), le corps de capsule (2) est rempli avec une quantité mesurée d'une préparation pharmaceutique, de préférence poudreuse, le corps de capsule est ensuite fermé avec un capuchon de capsule (1).
